# EUROPEAN PATENT APPLICATION

(11) **EP 4 541 823 A1**
(43) Date of publication of application: **23.04.2025**
(21) Application number: 23822596.5
(22) Date of filing: 16.06.2023
(51) Int. Cl.: C07K 19/00, G01N 33/566, G01N 33/53, A61P 35/00

(54) **PHASE CHANGE ADJUSTING ELEMENT AND USE THEREOF**

(30) Priority: 16.06.2022 CN 202210689199
(71) Applicant: Tsinghua University, Beijing 100084 (CN)
(72) Inventor: LI, Pilong, Beijing 100084 (CN); XU, Weifan, Beijing 100084 (CN); LIU, Yuyan, Beijing 100084 (CN); ZHU, Yuting, Beijing 100084 (CN); XIE, Leiming, Beijing 100084 (CN)
(74) Representative: Hoffmann Eitle
(86) International application number: PCT/CN2023/000077
(87) International publication number: WO 2023/241024

(57) **Abstract**

Provided is a novel phase transition adjusting element, a part of which comprises a multivalent phase transition domain, and the remaining comprises at least two ligands, wherein at least one ligand is covalently connected to the part containing the multivalent phase transition structural domain, the remaining ligands are covalently linked to the part comprising the multivalent phase transition structural domain or to other ligands, and each of the at least two ligands can specifically bind to cell surface molecules corresponding thereto. After binding to cell surface molecules, the phase transition adjusting element can effectively enrich cell surface molecules by means of driving phase separation, and enhance the aggregation of cell surface molecules (such as receptor oligomerization), thereby regulating and controlling various cell physiological and biochemical activities, such as receptor downstream signal transmission, cell endocytosis, etc.

## Description

### Technical Field

The present invention relates to a novel phase transition adjusting element, which can, after binding to cell surface molecules, effectively enrich cell surface molecules by means of driving phase separation, and enhance the aggregation of cell surface molecules (such as receptor oligomerization), thereby regulating and controlling various cell physiological and biochemical activities, such as receptor downstream signal transmission, cell endocytosis, etc.

### Background Art

Cell surface molecules are common candidate drug targets, but most of them are ultimately failed in developing useful drugs. As an example, the death receptor 5 (DR5), a member of the tumor necrosis factor (TNF) receptor superfamily, is not yet druggable.

Studies have found that multivalent macromolecules can aggregate due to intermolecular or intramolecular interactions, thereby separating from the common solution phase surrounding the molecules to form an independent liquid phase enriched with the macromolecules. This process is called "liquid-liqud phase separation (LLPS, also referred to as "phase separation" herein) or "phase transition". A large number of small droplets enriched with the macromolecules exist in the liquid phase formed by phase separation, and the diameter of the droplets can reach several microns or even larger. Such highly recognizable small droplets are called "phase transition droplets".

The use of chemically synthesized multivalent ligands to induce or stabilize dimerization or oligomerization of specific receptors has been reported as a tool for studying the functions of cell surface molecules. For example, multivalent ligands based on complex macrocyclic peptide backbones were reported (Science, 1993, 262(5136): 1019-1024). However, the enhancement effect of such multivalent ligands on receptor downstream signaling is strictly limited by the valency of the ligands. Based on these, IgM Biosciences reported the use of natural multivalent antibodies to increase the oligomerization of receptor molecules, and for example, a pentameric DR5-binding IgM was obtained by transplanting the DR5-binding VH domain onto the backbone of a natural multivalent ligand IgM(Mol Cancer Ther; 20(12): 2483-2494). However, the receptor binding activity of this polymeric IgM strongly depended on the antibody valency as well, it also displayed a higher risk of systemic toxicity as it can only recognize a single target.

To date, there have been no reports of using multivalent ligands comprising a part of multivalent phase transition domains having phase transition adjusting functions, and there have been no reports of using such multivalent ligands to manipulate corresponding cell surface molecules, thereby improving useful properties (such as the drugability) of receptors and other cell surface molecules.

### Summary of the Invention

Through in-depth research, the inventors discovered that a multivalent phase transition adjusting element is obtained by covalently linking a part containing a multivalent phase transition domain that can drive liquid-liquid phase separation to a plurality of ligand molecules that can specifically bind to different candidate cell surface molecular targets. The use of the multivalent phase transition adjusting element can not only improve the specificity and selectivity for the candidate targets, but also, compared with ligand molecules that do not have phase separation adjusting function, the multivalent phase transition adjusting element of the present invention shows significantly enhanced cell surface molecular aggregation effect and/or improved signal pathway manipulation activity.

Therefore, in one aspect, the present invention provides a phase transition adjusting element, characterized in that the phase transition adjusting element comprises a part containing a multivalent phase transition domain, and the remaining includes at least two ligands, wherein at least one of the ligands is connected to the part containing the multivalent phase transition domain, and the remaining ligands are connected to the part containing the multivalent phase transition domain or to other ligands contained in the same phase transition adjusting element, and each of the at least two ligands can specifically bind to acell surface molecule corresponding thereto, wherein the cell surface molecules can be identical or different.

In a preferred embodiment, the part containing the multivalent phase transition domain is covalently linked to the at least one ligand or the ligands are covalently linked to each other, optionally via a peptide linker or a non-peptide linker, preferably a peptide linker.

In another preferred embodiment, the phase transition adjusting element of the present invention is a fusion protein including the part containing the multivalent phase transition domain and at least two ligands.

In one embodiment, the phase transition adjusting element provided by the present invention includes three or more ligands.

In another embodiment, the number of ligands in the phase transition adjusting element is 2, 3, 4, 5, 6, 7, 8, 9, 10 or more.

In one embodiment, after the ligand contained in the phase transition adjusting element of the present invention binds to one or more cell surface molecules recognized by the ligand, the aggregation degree of the one or more cell surface molecules is significantly improved compared with the case where the phase transition adjusting element of the present invention is not bound, or compared with the case where the cell surface molecules bound to other multivalent ligands with the same valence as the phase transition adjusting element of the present invention.

In a preferred embodiment, the multivalent phase transition domain in the phase transition adjusting element of the present invention is composed of at least two, such as three, four, five, six, seven, eight, nine or ten tandemly linked motifs, and the motifs may be identical or different.

In a more preferred embodiment, the multivalent phase transition domain in the phase transition adjusting element of the present invention is composed of at least one SUMO3 motif tandemly linked with at least one SIM motif, or is composed of at least one PRMH motif tandemly linked with at least one SH3 motif.

In a further preferred embodiment, the multivalent phase transition domain in the phase transition adjusting element is composed of one, two, three or four SUMO3 motifs and one, two, three or four SIM motifs linked tandemly, or is composed of one, two, three or four PRMH motifs and one, two, three or four SH3 motifs linked tandemly.

In a further preferred embodiment, the multivalent phase transition domain includes an amino acid sequence shown in SEQ ID NO: 18 or SEQ ID NO: 19, or includes an amino acid sequence that is at least 80%, 90%, 95%, or 99% identical to the sequence shown in SEQ ID NO: 18 or SEQ ID NO: 19. In another preferred embodiment, the multivalent phase transition domain consists of an amino acid sequence shown in SEQ ID NO: 18 or SEQ ID NO: 19, or consists of an amino acid sequence that is at least 80%, 90%, 95%, or 99% identical to the sequence shown in SEQ ID NO: 18 or SEQ ID NO: 19.

In another preferred embodiment, a ligand contained in the phase transition adjusting element of the present invention is capable of specifically binding to a tumor-associated antigen.

In a more preferred embodiment, the tumor-associated antigen is selected from the group consisting of CXC motif chemokine receptor 4 (CXCR4), hepatocyte growth factor receptor (c-Met or HGFR), epidermal growth factor receptor (EGFR), human epidermal growth factor receptor 2 (HER2) and prostate-specific membrane antigen (PSMA), fibroblast activation protein (FAP), carcinoembryonic antigen (CEA), folate receptor alpha (FolR1), melanoma-associated chondroitin sulfate proteoglycan (MCSP), p95HER2, EpCAM, HER3, CD30 or TPBG (5T4), CD19, CD79b, CD20, CD22, CD37, CD38, BCMA and GPRC5D

In another preferred embodiment, the other ligand contained in the phase transition adjusting element of the present invention is capable of specifically binding to a tumor necrosis factor receptor, preferably death receptor 5 (DR5) or Fas, more preferably death receptor 5 (DR5).

In another preferred embodiment, the ligand in the phase transition adjusting element of the present invention can be selected from peptide ligands or non-peptide ligands, and the peptide ligand can preferably be selected from one or more of the following groups consisting of: antibodies or their antigen-binding fragments, cytokines, growth factors, adhesion molecules, peptide hormones, or polypeptides randomly selected by phage display or yeast display that specifically bind to cell surface molecules. In a more preferred aspect, the antibody may be selected from a monoclonal antibody, a polyclonal antibody, a human antibody or a humanized antibody; the antigen-binding fragment may be selected from F(ab')₂, Fab, a single-chain variable fragment (scFv), a single-domain antibody fragment (VHH or nanobody); and the non-peptide ligand is preferably selected from one or more of the group consisting of a small molecule agonist or antagonist, an antisense oligonucleotide or a small interfering RNA (siRNA).

In a further preferred embodiment, one or more ligands in the phase transition adjusting element of the present invention are single-chain variable fragments.

In a more preferred embodiment, one ligand contained in the phase transition adjusting element of the present invention is a single-chain variable fragment including an amino acid sequence shown in SEQ ID NO: 21 or an amino acid sequence having at least 80%, 90%, 95%, 99% identity to the sequence shown in SEQ ID NO: 21, and/or the other ligand is a single-chain variable fragment including an amino acid sequence selected from SEQ ID NO: 20, SEQ ID NO: 22 or SEQ ID NO: 23 or an amino acid sequence having at least 80%, 90%, 95%, 99% identity to the sequence shown in SEQ ID NO: 20, SEQ ID NO: 22 or SEQ ID NO: 23. In other preferred embodiments, one ligand contained in the phase transition adjusting element of the present invention consists of an amino acid sequence shown in SEQ ID NO: 21, or an amino acid sequence having at least 80%, 90%, 95%, or 99% identity with the sequence shown in SEQ ID NO: 21, and/or the other ligand consists of any one selected from the amino acid sequence shown in SEQ ID NO: 20, SEQ ID NO: 22 or SEQ ID NO: 23, or an amino acid sequence having at least 80%, 90%, 95%, or 99% identity with the sequence shown in SEQ ID NO: 20, SEQ ID NO: 22 or SEQ ID NO: 23.

In a preferred embodiment, the phase transition adjusting element of the present invention may further comprise a tag that does not affect the functions of the ligand and the part including the multivalent phase transition domain, and/or a polypeptide that cleaves the tag.

In some embodiments, the tag is used to separate, purify, and identify the part containing the multivalent phase transition domain.

In some embodiments, the tag may be selected from a polyhistidine (Hisₓ) tag, a maltose binding protein (MBP) tag, a glutathione S-transferase (GST) tag, and a small ubiquitin-like modifier (SUMO) tag. In the phase transition adjusting element of the present invention, the tag may be linked to the N-terminal and/or C-terminal of the part containing the multivalent phase transition domain.

The phase transition adjusting element according to the present invention, comprises, or is consisted of, the amino acid sequence shown in SEQ ID NO: 24 or SEQ ID NO: 25, or an amino acid sequence having at least 80%, 90%, 95%, or 99% identity to the amino acid sequence shown in SEQ ID NO: 24 or SEQ ID NO: 25.

In another aspect, the present invention provides a method for screening phase transition adjusting element, the method includes the following steps:
Step A: generating a library including a plurality of candidate phase transition adjusting elements, wherein each of the candidate phase transition adjusting elements includes a part including a multivalent phase transition domain, and at least two ligands, wherein at least one ligand is covalently linked to the part including the multivalent phase transition domain, and the remaining ligands are covalently linked to the part including the multivalent phase transition domain or to other ligands, and each of the at least two ligands can specifically bind to a cell surface molecule corresponding thereto, respectively, and optionally, the part including the multivalent phase transition domain and the at least one ligand that contained in the same phase transition adjusting element are covalently linked to each other via a peptide linker, wherein the cell surface molecule specifically bound by the ligand of each candidate phase transition adjusting element can be the same or different;
Step B: using a reference that does not contain the multivalent phase transition domain as a negative control, and measuring the activity level of the candidate phase transition adjusting element and the negative control in generating phase transition droplets under the same conditions suitable for phase transition; and
Step C: selecting the candidate phase transition adjusting element that produces more phase transition droplets than the negative control in step B as the target phase transition adjusting element.

In another aspect, the present invention provides a phase transition adjusting element obtained by screening through the above method for screening a phase transition adjusting element.

In yet another aspect, the present invention provides a pharmaceutical composition, characterized in that the pharmaceutical composition includes the aforementioned phase transition adjusting element, and optionally a pharmaceutically acceptable carrier.

The aforementioned phase transition adjusting element can be combined with one or more substances capable of inhibiting the internalization of cell surface receptors into one preparation, or they can be formulated into separate preparations so that they can be administered simultaneously, separately or sequentially. Therefore, in yet another aspect, the present invention also relates to a product including a phase transition adjusting element of the present invention and one or more receptor internalization inhibitors for simultaneous, separate or sequential use as a combined preparation in the treatment of a disease.

In still another aspect, the present invention provides a method of treating a disease using any of the aforementioned phase transition adjusting elements or pharmaceutical compositions.

In some embodiments, the method of treating a disease of the present invention includes administering an effective amount of a phase transition adjusting element of the present invention or a pharmaceutical composition of the present invention to a subject in need thereof. In a preferred embodiment, the disease may be selected from the group consisting of chronic autoimmune disorders, inflammatory disorders, diseases associated with abnormal cell proliferation or abnormal cell apoptosis, sepsis or viral infection. In a more preferred embodiment, the disease associated with abnormal cell proliferation or abnormal cell apoptosis is cancer. In further preferred embodiments, the cancer is selected from lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid cancer) or prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt's lymphoma or mycosis fungoides (MF), or metastatic forms thereof.

In still another aspect, the present invention provides use of the aforementioned phase transition adjusting element or the aforementioned pharmaceutical composition in manufacturing a preparation for treating a disease. In a preferred embodiment, the disease may be selected from the group consisting of chronic autoimmune disorders, inflammatory disorders, diseases associated with abnormal cell proliferation or abnormal cell apoptosis, sepsis or viral infection. In a more preferred embodiment, the disease associated with abnormal cell proliferation or abnormal cell apoptosis is cancer. In further preferred embodiments, the cancer is selected from lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid cancer) or prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt's lymphoma or mycosis fungoides (MF), or metastatic forms thereof.

According to the present invention, a new type of phase transition adjusting element is provided. The element can increase the aggregation of the cell surface molecules on the cell membrane by driving phase separation after binding to these molecules, thereby enhancing the regulatory effect on the physiological and biochemical activities related to the aggregationSuch enhancement does not depend on the valence of the ligand contained in the adjusting element. Moreover, since the novel adjusting element contains more than two ligands, it can achieve more variable and refined targeting specificity as well as more powerful controlling performance based on the combination between different ligands.

More specifically, the present invention provides a phase transition adjusting element comprising a part containing a multivalent phase transition domain, and at least a first ligand and a second ligand linked thereto. Compared with a multivalent ligand without a multivalent phase transition domain and thus unable to induce phase separation, the phase transition adjusting element of the present invention significantly enhances the regulatory effect on cell surface molecules that interact with the first ligand and the second ligand.

### The Description of Drawings

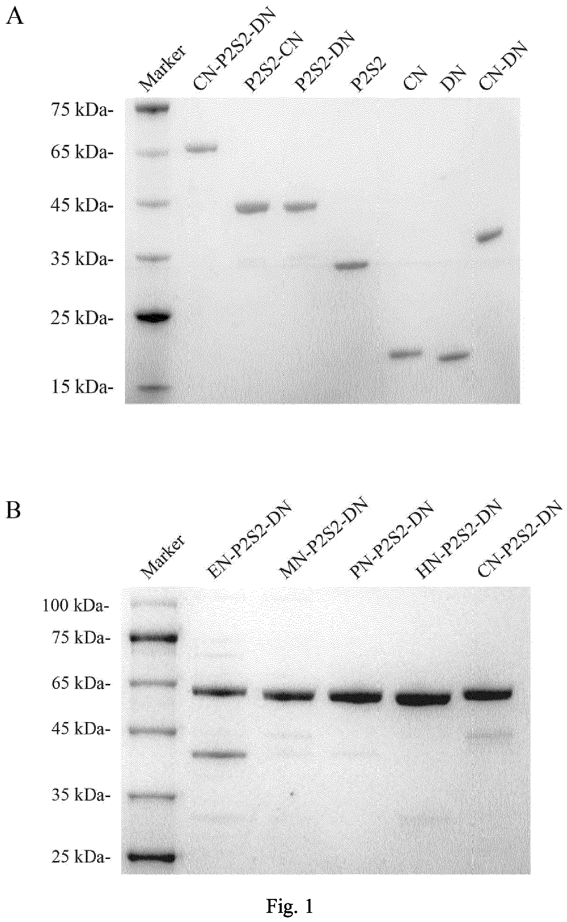

Fig. 1: Results of SDS-PAGE under reducing conditions for various synthetic ligands and the truncated variants thereof transiently expressed in E. coli BL21(DE3). Color development was performed by Coomassie Brilliant Blue staining. Fig. 1A is a graph showing the electrophoresis results of the synthetic ligand CN-P2S2-DN and its various truncated variants. Fig. 1B shows the SDS-PAGE results of synthetic ligands EN-P2S2-DN, MN-P2S2-DN, PN-P2S2-DN, HN-P2S2-DN and CN-P2S2-DN targeting different receptors. Marker indicates molecular weight marker (purchased from Beijing Juhemei Biotechnology Co., Ltd.).

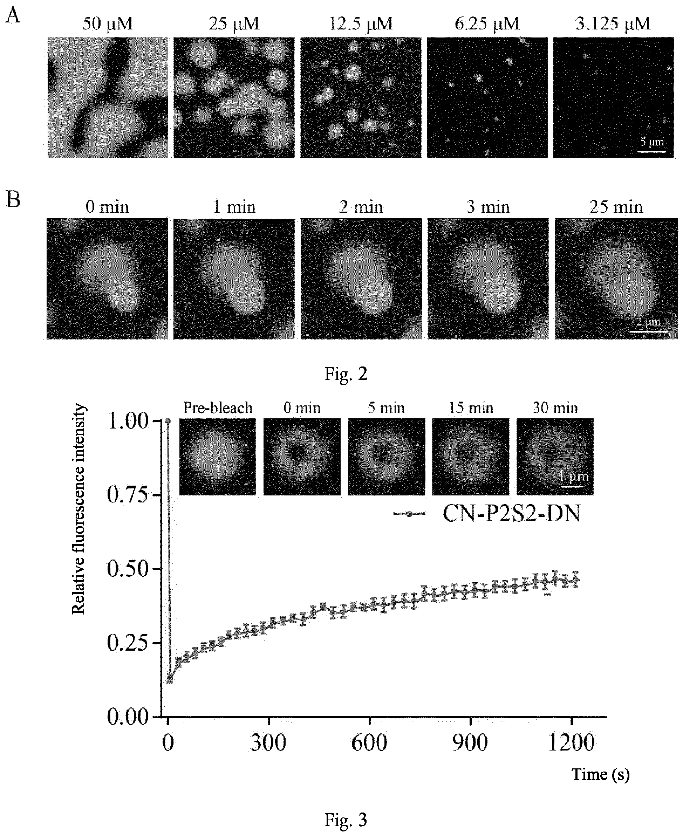

Fig. 2: Fig. 2A shows the laser confocal imaging results of an in vitro liquid-liquid phase separation experiment using the fluorescent dye Alexa 488-labeled CN-P2S2-DN protein at concentrations of 50 µM, 25 µM, 12.5 µM, 6.25 µM, and 3.125 µM, respectively. The scale bar in each panel is 5 µm. Fig. 2B shows the droplets formed by 12.5 µM CN-P2S2-DN protein in an in vitro phase separation experiment and their fusion process over time, which was captured using a NIKON A1R HD25 laser confocal microscope. 0 min indicates the initial time point of the observation time course.
Fig. 3: Fig. 3 shows the fluorescence recovery results after photobleaching (FRAP) of droplets formed by 12.5 µM CN-P2S2-DN protein in an in vitro phase separation experiment, photographing by a NIKON A1R HD25 laser confocal microscope. The upper figure is an imaging snapshot reprsenting the droplet changing over time; the lower figure is the corresponding statistical analysis result of fluorescence intensity. Time 0 represents the initial time point of observation and measurement immediately after the photobleaching pulsestimulation. The data points in the lower figure represent the average values of three independent experiments. The vertical axis shows the average fluorescence intensity after normalization and correction of the photobleached area based on the average fluorescence intensity of the unbleached area, and the horizontal axis shows the elapsed time (s) after the photobleaching pulse stimulation.

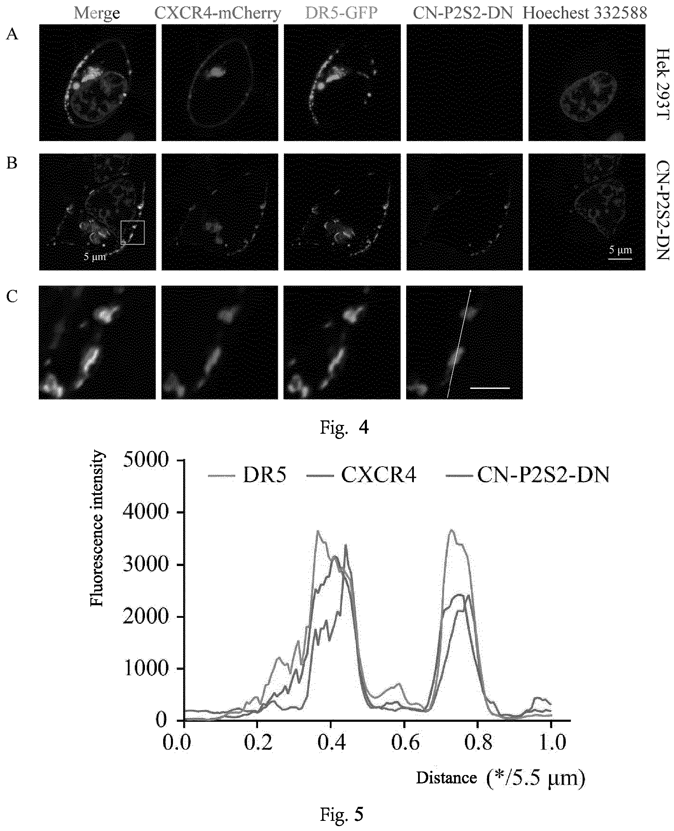

Fig. 4: Fig. 4 shows snapshots of laser confocal microscopy live cell imaging of normal HEK293T cells (as a control) and HEK293T cells co-expressing CXCR4-mCherry and DR5-GFP, both cells were treated with 200 nM CN-P2S2-DN protein (labeled with fluorescent dye Alexa 647) . Wherein, CXCR4-mCherry, DR5-GFP, CN-P2S2-DN and DAPI respectively show the fluorescence excitation images of exogenous CXCR4, exogenous DR5, CN-P2S2-DN proteins and cell nuclei, and Merge is the result of in situ overlapping the above four images. The scale bars in each panel are 5 µm. Fig. 4A shows HEK293T cells with the addition of a control solution. Fig. 4B shows HEK293T cells with the addition of CN-P2S2-DN protein. Fig. 4C is an enlarged view of the white dashed box area in Fig. 4B, showing the live cell imaging results of co-localization analysis of fluorescent signals representing CN-P2S2-DN, CXCR4 and DR5 in the puncta marked by white arrows. The scale bars in each panel are 1 µm.
Fig. 5: Gray value statistics of the fluorescence signal at the arrow mark in Fig. 4C. In the figures, the horizontal axis represents the relative distance along the direction indicated by the arrow, and the vertical axis represents the relative fluorescence intensity measured at the corresponding position.

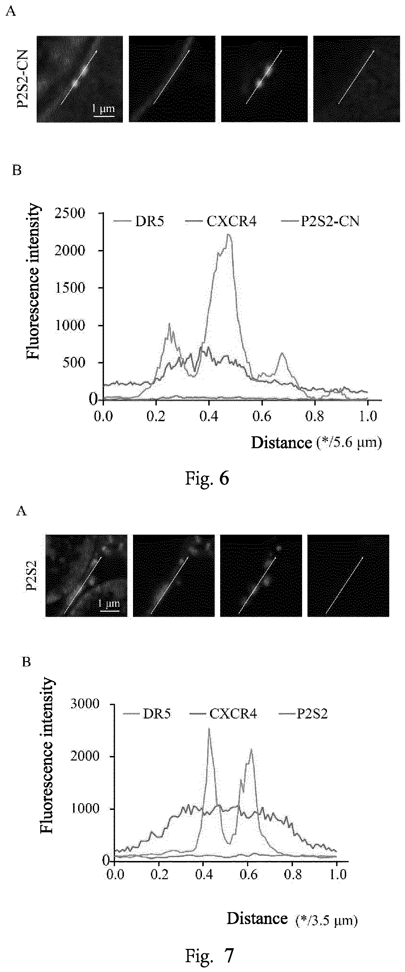

Fig. 6: Live cell imaging co-localization analysis results of the truncated variant P2S2-CN of CN-P2S2-DN and the exogenous receptors CXCR4-mCherry and DR5-GFP expressed on the surface of HEK293T cells. Fig. 6A is a fluorescence colocalization image of 200 nM P2S2-CN (labeled with Alexa 647) with CXCR4-mCherry and DR5-GFP, photographed by a NIKON A1R HD25 laser confocal microscope. The scale bars in each panel are 1 µm. Fig. 6B is the statistical result of the gray value of the fluorescence signal measured at the point marked by the white arrow. The horizontal axis in the figure represents the relative distance along the direction indicated by the arrow mark, and the vertical axis represents the relative fluorescence intensity measured at the corresponding position. Each data point represents the average of three independent experiments.
Fig. 7: Live cell imaging co-localization analysis results of the truncated variant P2S2 with exogenous receptors CXCR4-mCherry and DR5-GFP on the surface of HEK293T cells. Fig. 7A is the fluorescence colocalization images of 200 nM P2S2 (Alexa 647 labeled) with CXCR4-mCherry and DR5-GFP, photographed by a NIKON A1R HD25 laser confocal microscope. The scale bars in each panel are 1 µm. Fig. 7B is the statistical result of the gray value of the fluorescence signal measured at the point marked by the white arrow. The horizontal axis in the figure represents the relative distance along the direction indicated by the arrow mark, and the vertical axis represents the relative fluorescence intensity measured at the corresponding position. Each data point represents the average of three independent experiments.

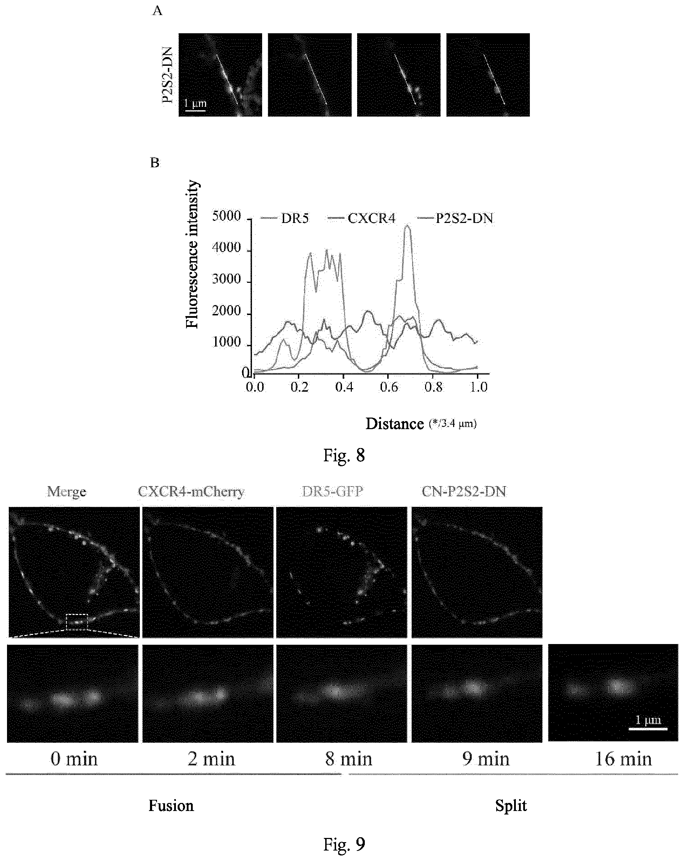

Fig. 8: Live cell imaging co-localization analysis results of the truncated variant P2S2-DN with exogenous receptors CXCR4-mCherry and DR5-GFP on the surface of HEK293T cells. Fig. 8A is the fluorescence colocalization image of 200 nM P2S2-DN (labeled with Alexa 647) with CXCR4-mCherry and DR5-GFP, photographed by a NIKON A1R HD25 laser confocal microscope. The scale bars in each panel are 1 µm. Fig. 8B is the statistical result of the gray value of the fluorescence signal measured at the point marked by the white arrow. The horizontal axis in the figure represents the relative distance along the direction indicated by the arrow mark, and the vertical axis represents the relative fluorescence intensity measured at the corresponding position. Each data point represents the average of three independent experiments.
Fig. 9: Time sequence results of laser confocal microscopy imaging of HEK293T cells co-expressing CXCR4-mCherry and DR5-GFP after treatment with 200 nM CN-P2S2-DN protein (labeled with fluorescent dye Alexa 647). CXCR4-mCherry, DR5-GFP and CN-P2S2-DN are fluorescence images showing exogenous CXCR4, exogenous DR5 and CN-P2S2-DN proteins, respectively, and Merge shows the result of in situ overlapping of the above three images. The upper panels are fluorescent images of intact cells. The scale bars in each panel are 5 µm. The lower figure is an enlarged view of the image within the white dotted box in the upper figure, showing the fusion and fission of puncta formed by the binding of CN-P2S2-DN to cell surface receptors CXCR4 and DR5 over time. The scale bars in each panel are 1 µm.

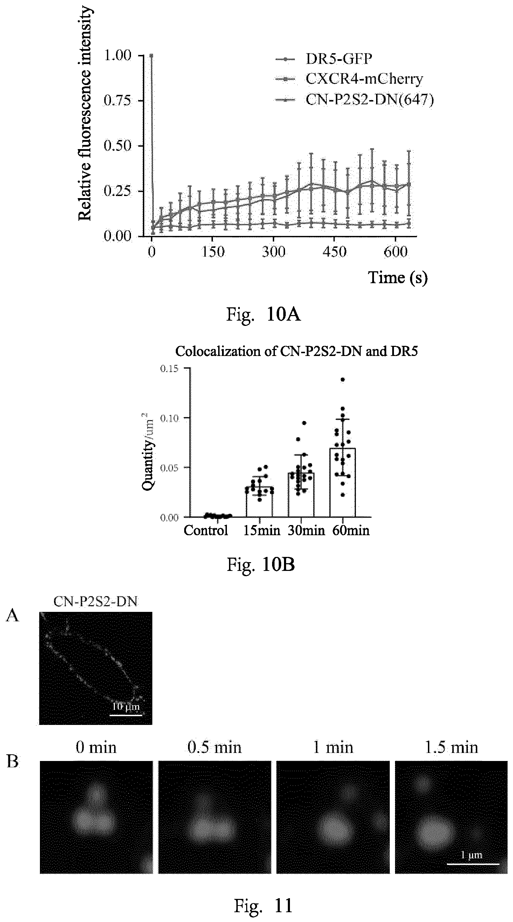

Fig. 10: Dynamic interaction of recombinant ligand CN-P2S2-DN with cell surface receptors. Fig. 10A shows the time sequence statistical analysis results of fluorescence recovery of CN-P2S2-DN, CXCR4-mCherry and DR5-GFP on the cell surface after FRAP was performed on HEK293T cells treated with CN-P2S2-DN (labeled with fluorescent dye Alexa 647). Each data point represents the mean of three independent experiments. The vertical axis of the graph represents the average fluorescence intensity of the photobleached area after normalization correction based on the average fluorescence intensity of the unbleached area, and the horizontal axis represents the elapsed time (s) after the photobleaching pulse stimulation. Fig. 10B shows the quantitative analysis results of the number of co-localized spots of CN-P2S2-DN and DR5. The X-axis shows the time since the addition of the recombinant ligand, and the Y-axis shows the number of spots having co-localization of CN-P2S2-DN and DR5 per square micron of the cell surface.
Fig. 11: Interaction of CN-P2S2-DN with endogenous receptors on the surface of SJSA-1 cells. Fig. 11A is a snapshot of live cell imaging of SJSA-1 cells treated with 200 nM CN-P2S2-DN protein (labeled with fluorescent dye Alexa 561), photographed by a NIKON A1R HD25 laser confocal microscope. The scale bar in the figure is 10 µm. Fig. 11B shows the time sequence results of live cell imaging of SJSA-1 cells treated with 200 nM CN-P2S2-DN protein (labeled with fluorescent dye Alexa 561), photographed bya NIKON A1R HD25 laser confocal microscope. The scale bar in the figure is 1 µm.

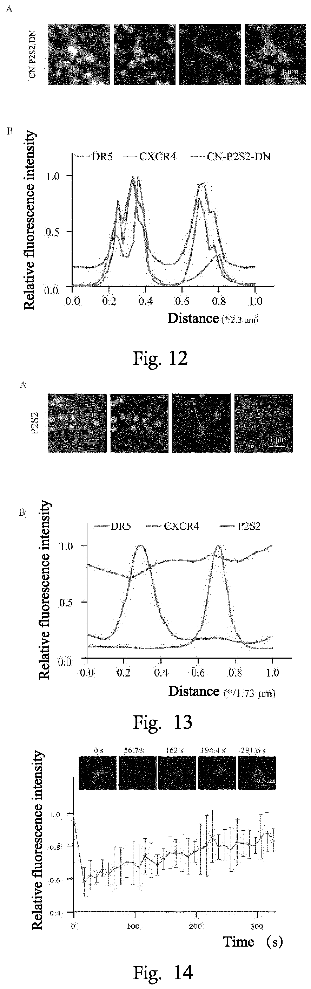

Fig. 12: Immunofluorescence co-localization analysis results of the synthetic ligand CN-P2S2-DN and the endogenous receptors CXCR4 and DR5 expressed on the surface of SJSA-1 cells. Fig. 12A is a fluorescence co-localization image of 200 nM CN-P2S2-DN (labeled with Alexa 561) and endogenous CXCR4 receptor and DR5 receptorc a NIKON A1R HD25 confocal microscope. The scale bars in each panel are 1 µm. Fig. 12B is the statistical analysis result of the gray value of the fluorescent signal measured at the straight line with a white arrow. In the figure, the horizontal axis represents the relative distance along the direction indicated by the arrow mark, and the vertical axis represents the relative fluorescence intensity measured at the corresponding position. Each data point represents the mean of three independent experiments.
Fig. 13: Immunofluorescence co-localization analysis results of the truncated variant P2S2 of CN-P2S2-DN with endogenous receptors CXCR4 and DR5 on the surface of SJSA-1 cells. Fig. 13A is a fluorescence co-localization image of 200 nM truncated variant P2S2 (labeled with Alexa 561) and endogenous CXCR4 receptor and DR5 receptor, photographed by a NIKON A1R HD25 confocal microscope. The scale bars in each panel are 1 µm. Fig. 13B is the statistical analysis result of the gray value of the fluorescent signal measured at the straight line with a white arrow. In the figure, the horizontal axis represents the relative distance along the direction indicated by the arrow, and the vertical axis represents the relative fluorescence intensity measured at the corresponding position. Each data point represents the mean of three independent experiments.
Fig. 14: Imaging snapshots of the fluorescence recovery of CN-P2S2-DN on the SJSA-1 cell surface after FRAP and the corresponding time sequence statistical analysis results,the cells were treated with 200 nM CN-P2S2-DN (labeled with fluorescent dye Alexa 561)and photographed by a NIKON A1R HD25 laser confocal microscope. Time 0 represents the initiating time point of observation and measurement immediately after the photobleaching pulse stimulation. The scale bars in the upper panels are all 0.5 µm. The vertical axis in the lower panel represents the average fluorescence intensity of the photobleached region after normalization correction using the average fluorescence intensity of the unbleached region as a reference, and the horizontal axis represents the elapsed time (s) after the photobleaching pulse stimulation. Each data point represents the mean of three independent experiments.

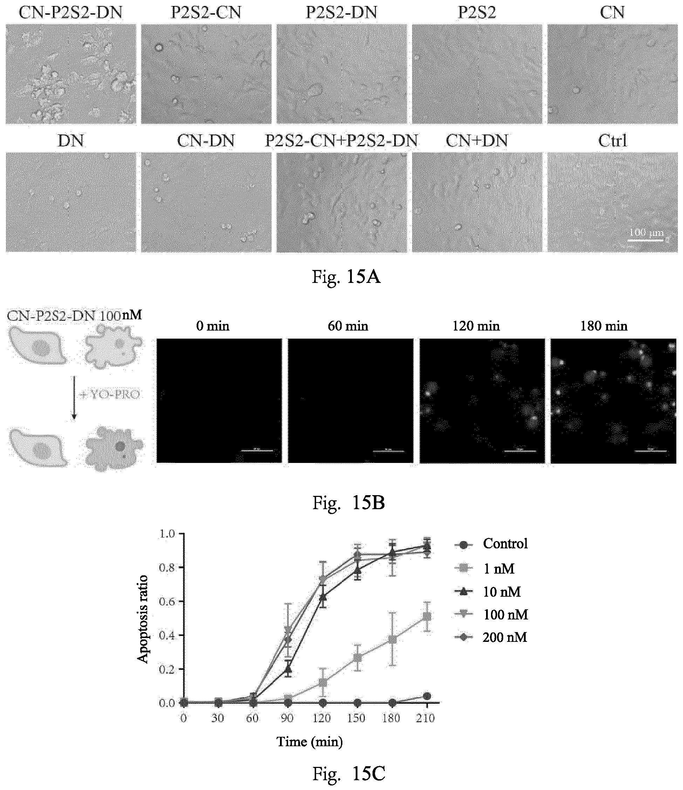

Fig. 15: Results of cell morphological changes observed after adding recombinant proteins, their truncated variants, or their combination. Fig. 15A shows the imaging results obtained by using an Olympus IX83 fluorescence inverted microscope after SJSA-1 cells were treated with 100 nM CN-P2S2-DN protein, various truncated variants thereof, or a combination of truncated variants for 3 hrs. Fig. 15B shows the imaging results of the nucleic acid dye YO-PRO entering the cells, photographed by a NIKON A1R HD25 laser confocal microscope at different time points after the treatment of 100 nM CN-P2S2-DN protein. Fig. 15C shows the imaging results photographed by a NIKON A1R HD25 laser confocal microscope at different CN-P2S2-DN addition concentrations and at different time points, and the time-dependent change curve of the proportion of YO-PRO fluorescence-positive cells measured by Image J (National Institutes of Health) and Graphpad Prism 8.

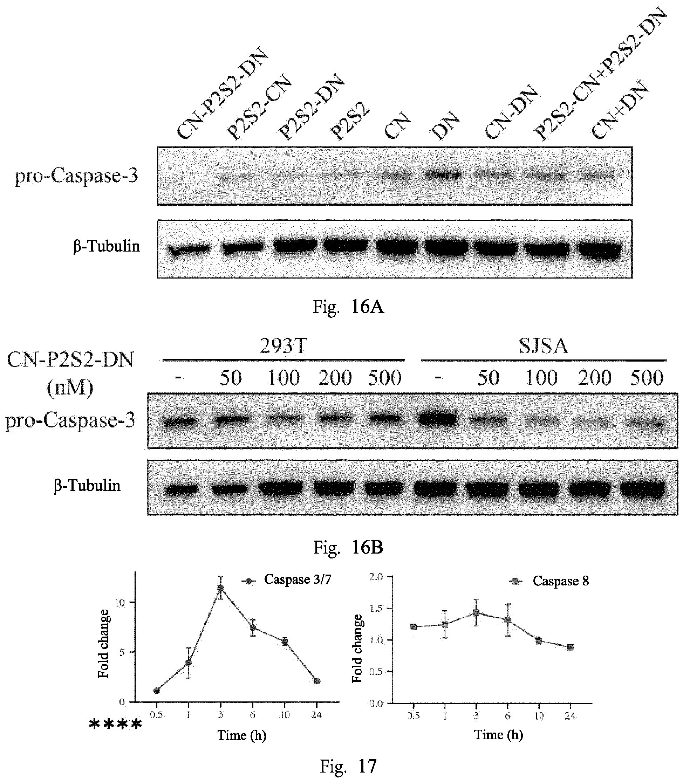

Fig. 16: Signal transduction pathways are altered in cells treated with recombinant proteins. Fig. 16A is a Western Blot analysis result of cysteinyl aspartate proteinase-3 (caspase-3) expressed in treated SJSA-1 cells. Fig. 16B is the results of Western immunoblotting analysis of caspase-3 expressed in HEK293T cells and SJSA-1 cells treated with different concentrations of CN-P2S2-DN protein. The cells were labeled with 0.1 µg/mL anti-Caspase-3 rabbit polyclonal antibody, and β-tubulin was used as an internal control.
Fig. 17: The kinetic curves over time of caspase-3/7 and caspase-8 were measuredby using Caspase-Glo^{®}.

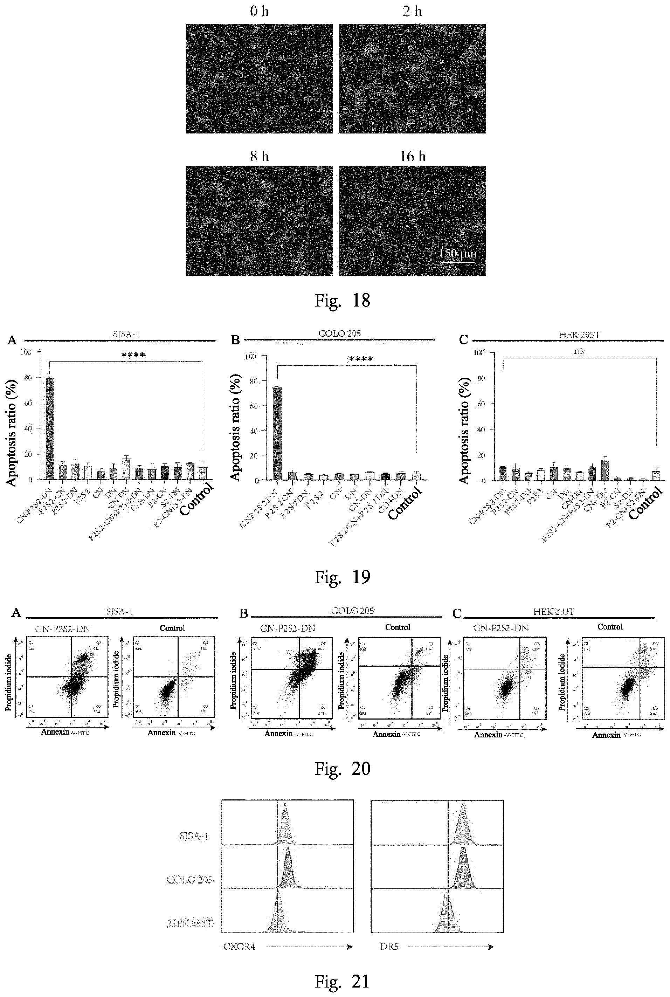

Fig. 18: Effects of 100 nM CN-P2S2-DN protein treatment on cell proliferation of SJSA-1 cells over time. SJSA-1 cells were labeled with CellTracker^{™} Green CMFDA Dye and monitored by the Incucyte^{®} Zoom System.
Fig. 19: Evaluation results of apoptosis-inducing activity of CN-P2S2-DN protein, various truncated variants thereof, and combinations of truncated variants on SJSA-1 cells (Fig. 19A), COLO205 cells (Fig. 19B), and 293T cells (Fig. 19C). The cells were treated with 100 nM CN-P2S2-DN protein, its truncated variant protein, or a combination thereof for 3 hr, and the percentage of cell apoptosis was determined by flow cytometry based on the Annexin V-FITC/PI double staining method. Ctrl in the figures represents blank solution control.
Fig. 20: Comparison of the apoptosis-inducing activity of CN-P2S2-DN protein on SJSA-1 cells, COLO-205 cells and HEK293T cells. Under the same conditions, SJSA-1 cells, COLO-205 cells and HEK293T cells were treated with 100 nM CN-P2S2-DN protein for 3 hr, and the percentages of SJSA-1 cells, COLO-205 cells and HEK293T cells distributed in different quadrants were determined by flow cytometry based on Annexin V-FITC/PI double staining. Ctrl in the figures represents blank solution control.
Fig. 21: shows the results of flow cytometric analysis of the expression levels of DR5 and CXCR4 in SJSA-1 cells, COLO-205 cells and HEK293T cells.

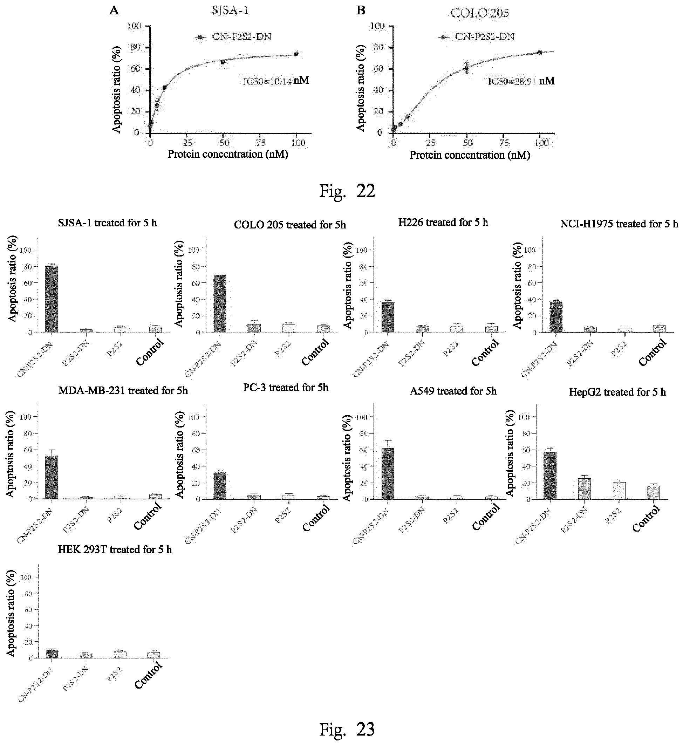

Fig. 22: Evaluation results of the apoptosis-inducing activity of CN-P2S2-DN protein on SJSA-1 cells and COLO-205 cells. SJSA-1 and COLO-205 cells were treated with different concentrations of CN-P2S2-DN protein solution, and cell apoptosis was determined by flow cytometry based on Annexin V-FITC/PI double staining method. The horizontal axis represents the concentration of the CN-P2S2-DN protein solution used, and the vertical axis represents the percentage of cell apoptosis.
Fig. 23: Evaluation results of the apoptosis-inducing activity of CN-P2S2-DN protein on different tumor cell lines and HEK293T cells. The cells were treated with 200 nM CN-P2S2-DN protein or its truncated variant protein for 5 hr, and then the apoptotic percentage of the treated cells was determined by flow cytometry based on Annexin V-FITC/PI double staining method. HEK293T cells were used as negative control in the figure.

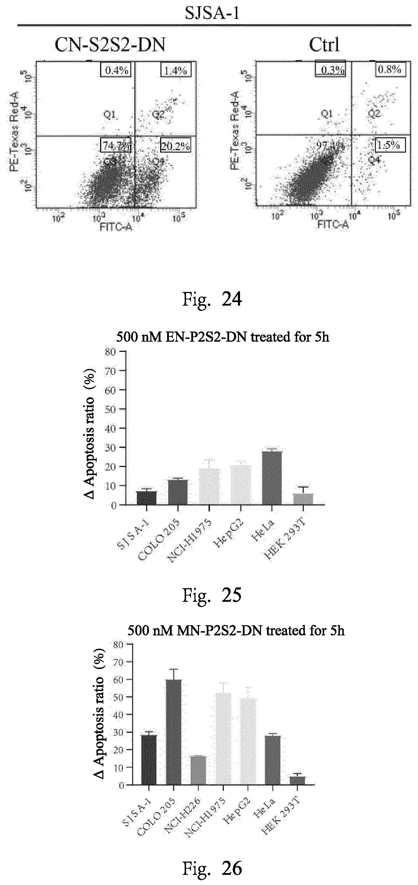

Fig. 24: Comparison of the apoptosis-inducing activity of CN-S2S2-DN protein on SJSA-1 cells and negative control cells. Under the same conditions, SJSA-1 cells and control cells were treated with 100 nM CN-S2S2-DN protein for 3 hr, and then the percentage of SJSA-1 cells and control cells distributed in different quadrants was determined by flow cytometry based on Annexin V-FITC/PI double staining. Ctrl in the figures represents HEK293T cells as a negative control.
Fig. 25: Comparison of apoptosis-inducing activity of EN-P2S2-DN protein on five tumor cell lines and HEK293T cells. 500 nM The cells were treated with EN-P2S2-DN for 5 hr and stained with Annexin V-FITC/PI double staining method.
Fig. 26: Comparison of apoptosis-inducing activity of MN-P2S2-DN protein on 6 tumor cell lines and HEK293T cells. The cells were treated with 500 nM MN-P2S2-DN for 5 hr and stained with Annexin V-FITC/PI double staining method.

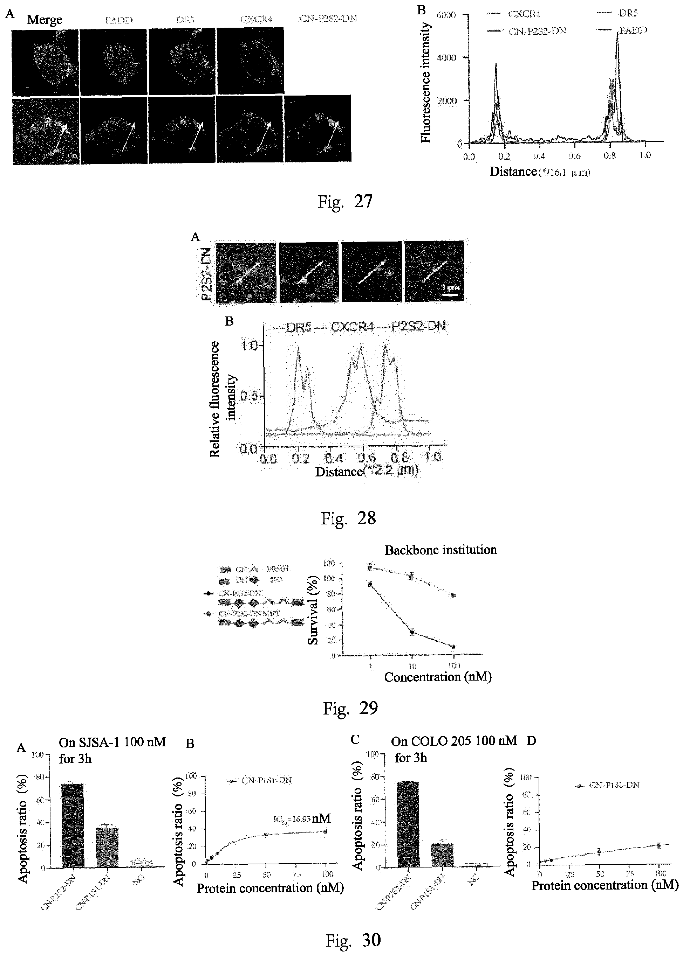

Fig. 27: Immunofluorescence co-localization analysis results of the synthetic ligand CN-P2S2-DN with exogenous receptors and their downstream signaling molecules in HEK293T cells. Fig. 27A is a fluorescence co-localization image of 200 nM CN-P2S2-DN (labeled with Alexa 647) and exogenous CXCR4 receptor, exogenous DR5 receptor, and exogenous FADD molecule, photographed by a NIKON A1R HD25 confocal microscope. The scale bars in each panel are 5 µm. Fig. 27B is the statistical analysis result of the gray value of the fluorescent signal measured at the straight line with a white arrow. In the figure, the horizontal axis represents the relative distance along the direction indicated by the arrow, and the vertical axis represents the relative fluorescence intensity measured at the corresponding position. Each data point represents the mean of three independent experiments.
Fig. 28: Results of immunofluorescence co-localization analysis of the truncated variant (P2S2-DN) of CN-P2S2-DN and the endogenous receptors CXCR4 and DR5 on the surface of SJSA-1 cells. Fig. 28A is a fluorescence co-localization image of 200 nM truncated variant P2S2-DN (labeled with Alexa 561) and endogenous CXCR4 receptor and DR5 receptor, photographed by a NIKON A1R HD25 confocal microscope. The scale bars in each panel are 1 µm. Fig. 28B is the statistical analysis result of the gray value of the fluorescent signal measured at the straight line with a white arrow. In the figure, the horizontal axis represents the relative distance along the direction indicated by the arrow, and the vertical axis represents the relative fluorescence intensity measured at the corresponding position. Each data point represents the mean of three independent experiments.
Fig. 29: Schematic diagram of the recombinant protein CN-P2S2-DN and its mutant CN-P2S2-DN MUT (left), and the apoptosis curves after adding the each of the proteins to tumor cells respectively (right).
Fig. 30: Evaluation results of the apoptosis-inducing activity of low-valent recombinant proteins on SJSA-1 cells and COLO-205 cells. Figs. 30A and 30C show the results of evaluating the apoptosis-inducing activity of the CN-P1S1-DN protein on two tumor cell lines. NC in the figures represents blank solution control. Figs. 30B and 30D show that SJSA-1 and COLO-205 cells were treated with different concentrations of CN-P1S1-DN protein solutions, and cell apoptosis was measured by flow cytometry based on Annexin V-FITC/PI double staining. The horizontal axis represents the concentration of the CN-P1S1-DN protein solution used, and the vertical axis represents the percentage of cell apoptosis.

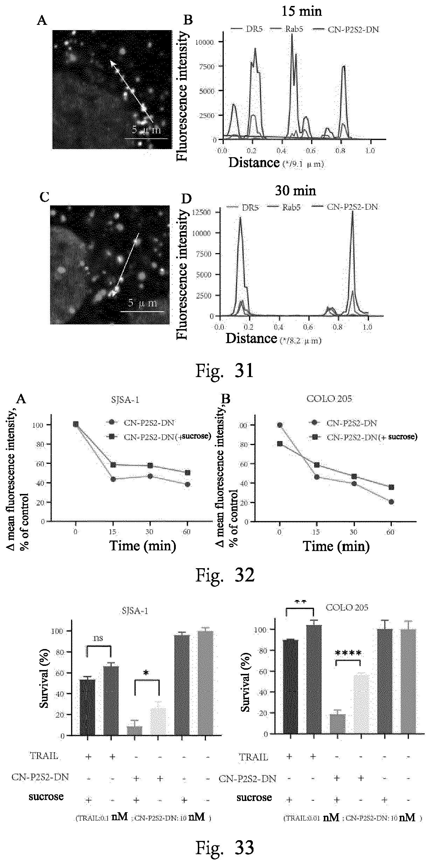

Fig. 31: Immunofluorescence analysis results of endogenous receptor DR5 (labeled with Alexa 488) and early endosomal marker Rab5 (labeled with Alexa 546) expressed on the surface of CN-P2S2-DN and SJSA-1 cells. Figs. 31A and 31C are fluorescence co-localization images of CN-P2S2-DN (labeled with Alexa 647) with DR5 and Rab5 taken at 15 minutes and 30 minutes, respectively, using a NIKON A1R HD25 laser confocal microscope. The scale bars in each panel are 5 µm. Figs. 31B and 31D are the statistical results of the grayscale values of the fluorescent signals measured at the points marked by white arrows, respectively. The horizontal axis in the figure represents the relative distance along the direction indicated by the arrow mark, and the vertical axis represents the relative fluorescence intensity measured at the corresponding position. Each data point represents the average of three independent experiments.
Fig. 32: The recombinant protein CN-P2S2-DN was added to cells pretreated with or without sucrose, and the time course curve of cell surface DR5 expression was observed. Fig. 32A shows SJSA-1 cells, and Fig. 32B shows COLO-205 cells. The X-axis represents different time points, and the Y-axis represents the average fluorescence intensity of DR5 measured by flow cytometry.
Fig. 33: The results of adding recombinant protein CN-P2S2-DN protein or TRAIL protein to cells pretreated with or without sucrose, and evaluating the survival rate of SJSA-1 cells or OLO205 cells, respectively. In the figure, ns means no statistically significant difference, * means p < 0.1, ** means p < 0.01, and **** means p < 0.0001.

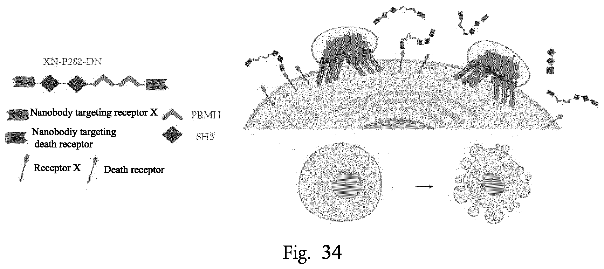

Fig. 34 is a schematic diagram of the cell apoptosis inducing mechanism of the phase transition adjusting element of the present invention.

### Detailed Description of the Preferred Embodiment

### Definition

As used in the description of the present invention, the following words and phrases are generally deemed to have the meanings set forth below, unless otherwise indicated in the context in which the words or phrases are used.

As used herein, the terms "comprise" or "include" mean that the compositions and methods include the recited elements, but do not exclude other elements. "Consisting essentially of," when used in defining compositions and methods, shall mean excluding any other components that are clearly essential to the combination. Therefore, the compositions defined herein consisting essentially of these components will not exclude trace contamination caused by separation and purification procedure or pharmaceutically acceptable carriers such as phosphate-buffered saline, preservatives and the like. "Consisting of" shall be meant to exclude minor components of other ingredients and substantial methods of administering the compositions of the present invention. Embodiments defined by these provisional terms are within the scope of the present invention.

As used herein, the term "about" refers to the common error range of the corresponding value that is readily known to those skilled in the art. A value or parameter described herein as "about" includes the value or parameter itself.

As used herein, the term "liquid-liquid phase separation" (LLPS, also referred to herein as "phase separation" or "phase transition") refers to the following transformation process that occurs between a multivalent macromolecule and its multivalent ligand: under suitable solution conditions, the multivalent macromolecule and its multivalent ligand aggregate through interaction to form a larger complex, and the complex separates from the common solution phase after reaching its solubility and forming an independent liquid phase enriched with the complex.

The term "phase transition droplets" refers to highly identifiable small droplets with a diameter of several microns or even larger that exist in the liquid phase formed by phase separation. "Phase transition droplets" herein are sometimes simply referred to as "droplets".

As used herein, the term "phase transition adjusting element" refers to a chemical entity that is capable of regulating cell surface molecules through phase separation, thereby regulating a series of cellular events associated with the cell surface molecules. For example, the phase transition adjusting element recruits the cell surface molecules bound to it into the phase transition condensate through the multivalent phase transition domain it contains, significantly increasing the local concentration of the cell surface molecules, thereby achieving the manipulation of various cellular events related to the cell surface molecules, including but not limited to recognizing external stimuli and promoting receptor downstream signal transduction, participating in enzymatic reactions, endocytosis, cell proliferation and differentiation, intercellular communication, etc.

As used herein, the term "multivalent phase transition domain" refers to a domain including multiple structural modules or motifs associated with phase separation. As mentioned above, biomacromolecules can aggregate and phase separate due to intermolecular or intramolecular interactions. Modules or motifs that can lead to the above-mentioned intermolecular or intramolecular interactions include, but are not limited to, (1) structural modules or motifs that are linearly arranged and have similar functions in proteins or polypeptides; (2) structural modules or motifs that promote oligomerization of proteins or polypeptides; (3) multimerization binding sites generated based on post-translational modifications; and (4) intrinsically disordered regions or low-complexity domains in proteins or polypeptides. See, e.g., Wang et al., Cell 174(3): 688-699, 2018; Nott, Timothy J et al., Molecular Cell 57(5): 936-947, 2015. The number of structural modules or motifs contained in the multivalent phase transition domain of the present invention that are essential for causing the above-mentioned intermolecular or intramolecular interactions is the valency of the multivalent phase transition domain. For example, for the multivalent phase transition domain P2S2 formed by two PRMH motifs and two SH3 motifs linkedtandem, the valency thereof is four.

In a preferred aspect, the multivalent phase transition domain of the present invention is formed by tandemly linking one or more SUMO3 motifs to one or more SIM motifs, or is formedby tandemly linking one or more PRMH motifs to one or more SH3 motifs. More preferably, the multivalent phase transition domain comprises an amino acid sequence shown in SEQ ID NO: 1 or SEQ ID NO: 12, or comprises an amino acid sequence having more than 80%, for example, 80%, 90%, 95%, 99% sequence identity with the sequence shown in SEQ ID NO: 1 or SEQ ID NO: 12, as long as the sequence can still induce phase separation of the adjusting element.

The term "ligand" herein is used in the broadest sense to refer to the other party in a pair that has a specific interaction with each other. More specifically, the term "ligand" means a chemical entity capable of interacting with a cell surface molecule. The interaction between the ligand involved in the present invention and the cell surface molecule it targets includes covalent bonds, ionic bonds, hydrogen bonds or other bonding methods known in the art. Relative to the interacting cell surface molecule, the ligand of the present invention may be naturally occurring, such as a natural molecule isolated from a target cell population or organism from which the cell surface molecule originates, such as certain small molecule metabolites, or macromolecules such as antibodies. The ligands of the present invention may also be non-natural, such as chemical entities obtained by artificial synthesis or modification that are different from natural molecules. That is to say, the term "ligand" herein encompasses various ligand structures, including but not limited to small molecules, antibodies, antigen-binding fragments, peptides, peptide mimetics, antisense oligonucleotides or small interfering RNA (siRNA), as long as they exhibit the desired specific binding activity with the pairing object. Preferably, the ligand herein refers to a peptide or peptide mimetic composed of natural or non-natural amino acids linked by peptide bonds, for example, an antibody or an antigen-binding fragment thereof, a cytokine, a growth factor, an adhesion molecule, a peptide hormone, and the like.

Herein, a "multivalent ligand" means that one molecule of ligand can bind to two molecules of or even more binding targets (eg, receptors), wherein the targets bound by the same molecule of multivalent ligand may be the same or different. The valence of a ligand refers to the number of binding regions contained in a macromolecule or its ligand that can interact with each other.

The term "cell surface molecule" herein has the meaning commonly understood in the art. Non-limiting examples of cell surface molecules include protein molecules, sugar molecules, glycoproteins, etc., receptors such as cytokine receptors, histocompatibility receptors, T cell receptors, etc., and ion channels. In some cases, the cell surface molecule may be a molecule that is present in the target cell population and substantially absent (or present at a lower concentration) in other populations, thereby being able to indicate or define the type of the target cell population. In other cases, the cell surface molecule may be a molecule present in the target cell population that is directly or indirectly associated with the state or condition of the pathology to be corrected and that is substantially absent or present at lower concentrations in cells or tissues not affected by the pathology. The ligand of the present invention can bind to the cell surface through interaction with cell surface molecules, preferably to the surface of cells having a pathological state or condition to be corrected (eg, tumor cells).

Herein, "receptor" may include endogenous receptors or exogenous receptors. Endogenous receptors include receptors that occur naturally in cells. Exogenous receptors include receptors that are introduced exogenously into a cell. In some aspects, the exogenous receptor may be a naturally occurring sequence contained in other cells of the same individual. In other aspects, the exogenous receptor can be a receptor from a different organism or a different species. Exogenous receptors also include synthetic receptors that do not occur naturally in any organism. Exogenous receptors include chimeric receptors, which refer to receptors constructed by connecting regions (e.g., extracellular, transmembrane, intracellular, etc. domains) of different molecules (e.g., different proteins, homologous proteins, orthologous proteins, etc.).

Herein, "tandemly linked" includes directly connecting the connection units through covalent bonds or indirectly connecting them through spacer sequences (also called linkers, connection sequences) and the like. In the tandemly linked structure, the connection units can be arranged in the same direction or in different directions.

The term "increase" or "activate" as used herein means the ability to cause an overall increase, e.g., an overall increase of 10% or more, 20% or more, 30% or more, 40% or more, 50% or more, or 75%, 85%, 90%, 95% or more. In certain aspects, increase or activation can refer to downstream activity of a ligand-cell surface molecule interaction.

The terms "reduce" or "inhibit" as used herein refer to the ability to cause an overall decrease, e.g., an overall decrease of 10% or more, 20% or more, 30% or more, 40% or more, 50% or more, or 75%, 85%, 90%, 95% or more. In certain aspects, reduction or inhibition can refer to an activity downstream of a ligand-cell surface molecule interaction.

As used herein, the term "oligomerization" means that several biomacromolecules, such as several receptor molecules, are aggregated into a complex by non-covalent bonds, and the functional state may be changed.

The term "antibody" herein encompasses various antibody structures including, but not limited to, monoclonal antibodies, polyclonal antibodies, multispecific antibodies (eg, bispecific antibodies), and antibody fragments (eg, bis-Fab).

The term "intact antibody" is used to refer to an antibody having a structure substantially similar to a native antibody structure or having heavy chains containing an Fc region as defined herein. Herein, "intact antibody" is used interchangeably with "full length antibody" and "whole antibody".

The term "antigen-binding fragment" or "antibody fragment" refers to a molecule other than an intact antibody that includes a part of an intact antibody that binds to the antigen to which the intact antibody binds. Examples of antigen-binding fragments include, but are not limited to, bis-Fab, Fv, Fab, Fab, Fab'-SH, F(ab')₂, diabodies, linear antibodies, single-chain antibodies (e.g., scFv, ScFab), single-domain antibodies (e.g., VH domain, VHH domain or nanobody), and multispecific antibodies formed from antibody fragments.

The term "single-chain antibody", also called "single-chain Fv", "single-chain variable fragment", "sFv" or "scFv", are antibody fragments including the VH and VL antibody domains connected in a single polypeptide chain. Preferably, the scFv polypeptide further includes a polypeptide linker between the VH and VL domains to enable the scFv to form a desired antigen binding structure. For a review of scFv, see Pluckthun, The Pharmacology of Monoclonal Antibodies, Vol. 113, Rosenburg and Moore, eds., Springer Verlag, New York, pp. 269-315 (1994); Malmborg et al., J. Immunol. Methods 183:7-13, 1995.

The term "single domain antibody" refers to an antibody fragment including all or part of the heavy chain variable domain or all or part of the light chain variable domain of an antibody. In certain aspects, the single domain antibody is a human single domain antibody (see, e.g., US Pat. No. 6,248,516 Bl). Examples of single domain antibodies include, but are not limited to, VHHs.

The term "small molecule" refers to any molecule having a molecular weight of about 2000 Daltons or less, such as about 1000 Daltons or less. In some aspects, the small molecule can be an organic molecule. In other aspects, the small molecule can be an inorganic molecule.

As used herein, the term "mimetic" or "molecular mimetic" refers to a polypeptide that has sufficient similarity in conformation and/or binding ability (e.g., secondary structure, tertiary structure) to a given polypeptide or a part of said polypeptide to bind to the binding partner of said polypeptide. A mimetic may bind to a binding partner with equal, lesser, or greater affinity than the polypeptide it mimics. A molecular mimetic may or may not have significant amino acid sequence similarity to the polypeptide it mimics. Mimetics can be naturally occurring or engineered. In some aspects, a mimetic can perform all of the functions of the polypeptide being mimicked. In other aspects, a mimetic does not perform all of the functions of the polypeptide it is mimicking.

As used herein, "tumor-associated antigen" or "TAA" refers to an antigenic determinant present on the surface of a target cell, eg, a cell in a tumor (such as a cancer cell, a cell in tumor matrix). In certain aspects, the target cell antigen is an antigen on the surface of a tumor cell. In one aspect, the TAA is selected from the group consisting of: CXC motif chemokine receptor 4 (CXCR4), hepatocyte growth factor receptor (c-Met or HGFR), epidermal growth factor receptor (EGFR), epidermal growth factor receptor 2 (HER2) and prostate specific membrane antigen (PSMA), fibroblast activation protein (FAP), carcinoembryonic antigen (CEA), folate receptor alpha (FolR1), melanoma-associated chondroitin sulfate proteoglycan (MCSP), p95HER2, EpCAM, HER3, CD30 or TPBG (5T4), CD19, CD79b, CD20, CD22, CD37, CD38, BCMA and GPRC5D.

Unless otherwise indicated, the term "CXC motif chemokine receptor 4" or "CXCR4" as used herein refers broadly to any native CXCR4 from any vertebrate source, including mammals such as primates (e.g., humans), non-human primates (e.g., cynomolgus monkeys), and rodents (e.g., mice and rats). The term encompasses full-length CXCR4 and isolated regions or domains of CXCR4, such as the extracellular domain of CXCR4. The term also encompasses naturally occurring variants of CXCR4, such as splice variants or allelic variants. An exemplary amino acid sequence of human CXCR4 is shown in Uniprot ID: P61073. The present invention also contemplates minor sequence variations, particularly conservative amino acid substitutions of CXCR4 that do not affect the function and/or activity of CXCR4.

Unless otherwise indicated, the term "death receptor 5" or "DR5" as used herein refers broadly to any native DR5 from any vertebrate source, including mammals such as primates (e.g., humans), non-human primates (e.g., cynomolgus monkeys), and rodents (e.g., mice and rats). The term encompasses full-length DR5 and isolated regions or domains of DR5, such as a DR5 extracellular domain. The term also encompasses naturally occurring variants of DR5, such as splice variants or allelic variants. An exemplary amino acid sequence of human DR5 is shown in Uniprot ID: 014763. The present invention also contemplates minor sequence variations, especially conservative amino acid substitutions of DR5 that do not affect the function and/or activity of DR5.

The term "epidermal growth factor receptor (EGFR)" is also known as proto-oncogene c-ErbB-1 or receptor tyrosine protein kinase erbB-1. Unless otherwise indicated, the term refers broadly to any native EGFR from any vertebrate source, including mammals such as primates (eg, humans), non-human primates (eg, cynomolgus monkeys), and rodents (eg, mice and rats). The term encompasses full-length EGFR and isolated regions or domains of EGFR, such as the EGFR extracellular domain. The term also encompasses naturally occurring variants of EGFR, such as splice variants or allelic variants. The amino acid sequence of human EGFR is shown in UniProt Accession No. P00533 (version 211). The present invention also contemplates minor sequence variations, especially conservative amino acid substitutions of EGFR that do not affect the function and/or activity of EGFR.

Unless otherwise indicated, the term "hepatocyte growth factor receptor (Met)" as used herein refers broadly to any native hepatocyte growth factor receptor from any vertebrate source, including mammals such as primates (e.g., humans), non-human primates (e.g., cynomolgus monkeys), and rodents (e.g., mice and rats). The term encompasses the full-length hepatocyte growth factor receptor as well as isolated regions or domains thereof, such as the extracellular domain of the hepatocyte growth factor receptor. The term also encompasses naturally occurring variants of the hepatocyte growth factor receptor, such as splice variants or allelic variants. The present invention also contemplates minor sequence variations, especially conservative amino acid substitutions of the hepatocyte growth factor receptor that do not affect the function and/or activity of the hepatocyte growth factor receptor.

As used herein, an "effective amount" refers to an amount effective, at dosages and for periods of time necessary, to achieve the desired result. The desired outcome may be, for example, alleviation of symptoms, prolonged survival, improved mobility, and the like. **In** preferred embodiments, the administration includes, but is not limited to, topical administration, parenteral administration, mucosal administration, intranasal administration, intraocular administration, intrathecal administration, subdural administration, subcutaneous administration, and enteral administration.

As used herein, "internalization" refers to the transfer of a cell surface protein to the interior of a cell through a process called internalization. Therefore, "receptor internalization inhibitor" refers to an agent that can inhibit, hinder, reduce or eliminate the receptor internalization process, including but not limited to any substance known in the art or not yet found to have internalization inhibitory activity. Receptor internalization inhibitors affect signal transduction and cell function by interfering with or hindering the internalization process of receptors and their bound signal molecules.

As used herein, "disease" refers to any condition that would benefit from treatment, including, but not limited to, chronic and acute disorders or diseases, including those pathological conditions that predispose a mammal to the disorder. In certain aspects, the disease is a disease associated with altered function or activity of a cell surface molecule. In certain preferred aspects, the disease is a chronic autoimmune disorder, an inflammatory disorder, a disease associated with abnormal cell proliferation, a disease associated with abnormal cell apoptosis, sepsis, or a viral infection. In the most preferred aspect, the disease is cancer.

The term "cancer" as used herein refers to or describes the physiological condition in mammals that is typically characterized by uncontrolled cell growth/proliferation. Cancer aspects include solid tumor cancers and non-solid tumor cancers. Solid cancer tumors include, but are not limited to, lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid carcinoma), or prostate cancer, or metastatic forms thereof. In some aspects, the cancer is colorectal cancer (CRC). In some aspects, the cancer is lung cancer. Further aspects of lung cancer include epidermal growth factor receptor positive (EGFR⁺) lung cancer. Other aspects of lung cancer include non-small cell lung cancer (such as squamous lung cancer or non-squamous lung cancer) and small cell lung cancer. In some aspects, the cancer is prostate cancer. Further aspects of prostate cancer include castration-resistant prostate cancer (CRPC). In some aspects, the cancer is breast cancer. Further aspects of breast cancer include HER2-positive (HER2⁺) breast cancer. Other aspects of breast cancer include ductal carcinoma. In some aspects, the breast cancer is early stage breast cancer. In some aspects, the cancer is a metastatic form of a solid tumor. In some aspects, the metastatic form of a solid tumor is a metastatic form of lung cancer, colorectal cancer, head and neck cancer, glioma, neuroblastoma, melanoma, breast cancer, bladder cancer, kidney cancer, ovarian cancer, pancreatic cancer, cervical cancer, esophageal cancer, sarcoma, esophageal cancer, thyroid cancer, and prostate cancer. In some aspects, the cancer is a non-solid tumor cancer. Non-solid tumor cancers include, but are not limited to, B-cell lymphomas. Further aspects of B-cell lymphoma include, for example, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt lymphoma or mycosis fungoides (MF).

The term "pharmaceutically acceptable carrier" refers to a non-toxic carrier that can be administered to a patient together with the phase transition adjusting element of the present invention without destroying the functional activity of the adjusting element. In certain embodiments, a "pharmaceutically acceptable" substance is suitable for use in contact with cells, tissues or organs of animals or humans without excessive toxicity, irritation, allergic response, immunogenicity or other adverse reactions, and the amount used in the dosage form is proportional to a reasonable benefit/risk ratio according to the administration schedule. In certain embodiments, a "pharmaceutically acceptable" substance as a component of a pharmaceutical composition is also compatible with the other ingredients of the composition. In certain embodiments, the term "pharmaceutically acceptable carrier" includes, but is not limited to, pharmaceutically acceptable inactive ingredients, materials, compositions and vehicles, such as liquid fillers, solid fillers, diluents, excipients, carriers, solvents and encapsulating materials. Carriers also include all pharmaceutically acceptable dispersion media, coatings, buffers, isotonic agents, stabilizers, absorption delaying agents, antimicrobial agents, antibacterial agents, antifungal agents, adjuvants, and the like. Unless any conventional carrier is incompatible with the phase transition adjusting element, the present disclosure contemplates use of conventional carriers in pharmaceutical compositions. See, e.g., Remington: The Science and Practice of Pharmacy, 21st ed., Lippincott Williams & Wilkins (Philadelphia, Pennsylvania, 2005); Handbook of Pharmaceutical Excipients, 5th Ed., Rowe et al., ed., The Pharmaceutical Press and the American Pharmaceutical Association (2005); Handbook of Pharmaceutical Additives, 3rd ed., Ash and Ash, ed., Gower Publishing Co. (2007); and Pharmaceutical Preformulation and Formulation, Gibson, ed., CRC Press LLC (Boca Raton, Florida, 2004).

The following describes the preferred modes for implementing the present invention. It should be noted that the embodiments described below are examples showing typical embodiments of the present invention, but the present invention is not limited to these examples.

Two or more of the embodiments described below may be combined, and such a combination is also included in the present invention.

### Examples

### Materials:

Fetal bovine serum: purchased from GIBCO, ThermoFisher Scientific
Pen-Strep: purchased from GIBCO, ThermoFisher Scientific
DMEM: purchased from HyClone, Cytiva
RMPI-1640: purchased from GIBCO, ThermoFisher Scientific

### Cell lines:

The cell lines used in this application were purchased from the China National Biomedical Experimental Cell Resource Bank ()

### A. HEK293T cell line

HEK293T human renal epithelial cell line was maintained in DMEM containing 10% fetal bovine serum and 100 units/ml Pen-Strep at 37°C and 5% CO₂.

### B. SJSA-1 cell line

The human osteosarcoma cell line SJSA-1 (formerly known as OsA-CL) was maintained in RMPI-1640 medium containing 10% fetal bovine serum and 100 Units/ml Pen-Strep at 37°C and 5% CO₂.

### C. COLO-205 cell line

Human colon adenocarcinoma cell line COLO-205 was maintained in DMEM containing 10% fetal bovine serum and 100 units/ml Pen-Strep at 37°C and 5% CO₂.

### D. PC-3 cell line

Human prostate adenocarcinoma cell line PC-3 was maintained in DMEM containing 10% fetal bovine serum and 100 units/ml Pen-Strep at 37°C and 5% CO₂.

### E. NCI-H226 cell line

Human lung squamous cell carcinoma cell line NCI-H226 was maintained in DMEM containing 10% fetal bovine serum and 100 units/ml Pen-Strep at 37°C and 5% CO₂.

### F. T-47D cell line

Human breast cancer ductal cell line T-47D was maintained in DMEM containing 10% fetal bovine serum, 0.2 Units/ml insulin (purchased from Life Technologies) and 100 Units/ml Pen-Strep at 37°C and 5% CO₂.

### G. THP-1 cell line

The human leukemic monocytic cell line THP-1 was maintained at 37°C, 5% CO₂ in RMPI-1640 medium containing 10% fetal bovine serum, 0.05 mM 2-mercaptoethanol (purchased from Sigma-Aldrich), and 100 Units/ml Pen-Strep.

### H. OCI-AML3 cell line

The human acute myeloid leukemia cell line OCI-AML3 was maintained in RMPI-1640 containing 20% fetal bovine serum and 100 units/ml Pen-Strep at 37°C and 5% CO₂.

### I. NCI-H1975 cell line

Human lung squamous cell carcinoma cell line NCI-H1975 was maintained in RMPI-1640 containing 10% fetal bovine serum and 100 units/ml Pen-Strep at 37°C and 5% CO₂.

### J. MDA-MB-231 cell line

The human breast epithelial carcinoma cell line MDA-MB-231 was maintained at 37°C, 5% CO₂ in F-12K (GIBCO) containing 10% fetal bovine serum and 100 Units/ml Pen-Strep.

### K. A549 cell line

The human lung cancer cell line A549 was maintained in DMEM (HyClone, Cytiva) containing 10% fetal bovine serum and 100 Units/ml Pen-Strep at 37°C and 5% CO₂.

### L. HepG2 cell line

The human hepatoma cell line HepG2 was maintained in MEM (GIBCO, Thermo Fisher Scientific) containing 10% fetal bovine serum and 100 Units/ml Pen-Strep at 37°C and 5% CO₂.

### N. Hela cell line

The human cervical cancer cell line Hela was maintained in DMEM containing 10% fetal bovine serum and 100 units/ml Pen-Strep at 37°C and 5% CO₂.

### Antibodies:

**Table 1: Antibodies used in the present invention**

| Antibody | Source | Identifier |
|---|---|---|
| Mouse anti-GAPDH monoclonal antibody | Abmart | Cat# M20006, RRID:AB_2737054 |
| Rabbit anti-β-tubulin polyclonal antibody | CST | Cat# 2146, RRID:AB_2210545 |
| Rabbit anti-DR5-specific polyclonal antibody | Proteintech | Cat# 15497-1-AP; RRID:AB_2240702 |
| Mouse anti-CXCR4 monoclonal antibody | Proteintech | Cat# 60042-1-Ig; RRID:AB_2091809 |
| Rabbit anti-FADD polyclonal antibody | Proteintech | Cat# 14906-1-Ap; RRID:AB_2100486 |
| Rabbit anti-Caspase-3 polyclonal antibody | CST | Cat# 9662, RRID:AB_331439 |
| Rabbit anti-C-Met monoclonal antibody | CST | Cat# 8198S; RRID: AB_10858224 |
| Rabbit anti-EGFR monoclonal antibody | CST | Cat# 4267; RRID: AB_2246311 |
| F(ab')₂ - goat anti-mouse IgG (H+L) cross-adsorbed secondary antibody, HRP | Thermo Fisher Scientific | Cat# A24524; RRID:AB_2535993 |
| F(ab')₂ -goat anti-rabbit IgG (H+L) cross-adsorbed secondary antibody, HRP | Thermo Fisher Scientific | Cat# A24537; RRID:AB_2536005 |
| Donkey anti-rabbit IgG (H+L) Highly cross-adsorbed secondary antibody, Alexa Fluor^{™} Plus 488 | Thermo Fisher Scientific | Cat# A32790; RRID: AB_2762833 |
| Donkey anti-mouse IgG (H+L) Highly cross-adsorbed secondary antibody, Alexa Fluor^{™} Plus 568 | Thermo Fisher Scientific | Cat# A10037; RRID: AB_2534013 |
| Rab5A (E6N8S) mouse mAb | Cell Signaling Technology | Cat# 46449, RRID:AB_2799303 |

### Example 1: Construction of expression vector

DNA molecules encoding the amino acid sequences shown in SEQ Nos. 1 to 13 and 16 to 17 in Table 2 below were artificially synthesized. The obtained DNA molecules were inserted into the pRSFDuet-1 vector (Novagen product of Merck), and the DNA fragments (including enzyme recognition sequences) between the NcoI and XhoI restriction enzyme site recognition sequences were replaced to obtain recombinant prokaryotic expression vectors.

Artificially synthesize DNA molecules encoding CXCR4-mCherry or DR5-GFP (as shown in sequence numbers 14-15 in Table 2 below). The obtained DNA molecules were inserted into the pcDNA3.1 vector (Invitrogen), and the DNA fragment (including the restriction enzyme recognition sequence) between the HindIII and XhoI restriction enzyme site recognition sequences was replaced to obtain the recombinant eukaryotic expression vectors pcDNA3.1-CXCR4-mCherry and pcDNA3.1-DRS-GFP, respectively. The plasmid was extracted using an endotoxin-free plasmid extraction kit (purchased from Jiangsu Kangwei Century Biotechnology Co., Ltd.) according to the manufacturer's instructions and stored at -20°C.

**Table 2: Constructs used in the present invention**

| Construct | Description | SEQ ID NO. |
|---|---|---|
| MBP-P2 S2-His₆ | Tandem repeats of the PRMH domain and SH3 domain fused with an MBP tag at the N-terminal and a His₆ tag at the C-terminal | 1 |
| MBP-CN-His₆ | Anti-CXCR4 nanobody fused with an MBP tag at the N-terminal and a His₆ tag at the C-terminal | 2 |
| MBP-DN-His₆ | Anti-DR5 nanobody fused with an MBP tag at the N-terminal and a His₆ tag at the C-terminal | 3 |
| MBP-EN-His₆ | Anti-EGFR nanobody fused with an MBP tag at the N-terminal and a His₆ tag at the C-terminal | 4 |
| MBP-MN-His₆ | Anti-C-Met nanobody fused with an MBP tag at the N-terminal and a His₆ tag at the C-terminal | 5 |
| MBP-CN-DN-His₆ | Fusion of CN and DN fused with an MBP tag at the N-terminal and a His₆ tag at the C-terminal | 6 |
| MBP-CN-P2S2-DN-His₆ | Fusion of CN, P2S2 and DN fused with an MBP tag at the N-terminal and a His₆ tag at the C-terminal | 7 |
| MBP-EN-P2S2-DN-His₆ | Fusion of EN, P2S2 and DN fused with an MBP tag at the N-terminal and a His₆ tag at the C-terminal | 8 |
| MBP-MN-P2S2-DN-His₆ | Fusion of MN, P2S2 and DN fused with an MBP tag at the N-terminal and a His₆ tag at the C-terminal | 9 |
| MBP-P2S2-CN-His₆ | Fusion of CN and P2S2 fused with an MBP tag at the N-terminal and a His₆ tag at the C-terminal | 10 |
| MBP-P2S2-DN-His₆ | Fusion of DN and P2S2 fused with an MBP tag at the N-terminal and a His₆ tag at the C-terminal | 11 |
| MBP-S2S2-His₆ | Tandem repeats of the SUMO3 domains and SIM domains fused with an MBP tag at the N-terminal and a His₆ tag at the C-terminal | 12 |
| MBP-CN-S2S2-DN-His₆ | Fusion of CN, S2S2 and DN fused with an MBP tag at the N-terminal and a His₆ tag at the C-terminal | 13 |
| CXCR4-mCherry | CXCR4 fused with an mCherry tag at the C-terminal | 14 |
| DR5-GFP | DR5 fused with a GFP tag at the C-terminal | 15 |
| P2-CN | Fusion of CN and P2 | 16 |
| S2-DN | Fusion of S2 and DN | 17 |
| P2S2 | Tandem repeats of two PRMH domains and two SH3 domains | 18 |
| S2S2 | Tandem repeats of two SUMO3 domains and two SIM domains | 19 |
| CN | Anti-CXCR4 Nanobody | 20 |
| DN | Anti-DR5 Nanobody | 21 |
| EN | Anti-EGFR Nanobody | 22 |
| MN | Anti-C-Met Nanobody | 23 |
| CN-P2S2-DN | Fusion of CN, P2S2 and DN | 24 |
| CN-S2S2-DN | Fusion of CN, S2S2 and DN | 25 |
| FADD-BFP | FADD fused with a BFP tag at the C-terminal | 26 |
| CN-P1S1-DN | Fusion of CN, P1S1 and DN | 27 |
| CN-P2S2-DN MUT | Fusion of CN, P2S2 (mutant) and DN fused with an MBP tag at the N-terminal and a His₆ tag at the C-terminal | 28 |

### Example 2: Expression and purification of recombinant protein

The prokaryotic expression vector prepared in Example 1 was purified using methods well known in the art, and the purified vector was transformed into *Escherichia coli* BL21 (DE3). See, for example, Maryam B, Hossein KS et al., Expression and Purification of Biologically Active Recombinant Rabbit Monocyte Chemoattractant Protein1 in Escherichia coli. FEMS Microbiology Letters, 2018(9):9.

The identified single clone was picked into LB liquid medium. Incubation at 37°C with shaking until DO₆₀₀ was approximately 0.8. After cooling to 18°C, 0.5 mM isopropyl β-D-thiogalactoside (IPTG, purchased from INALCO, USA) was added to induce expression for 16 hr. The pellet was collected by centrifugation at 4000 x g for 30 min, resuspended in a basic buffer containing 40 mM Tris-HCl (pH 7.4), 500 mM NaCl, and 10 mM imidazole, and disrupted by sonication. Then, the cells were centrifuged at 20,000 × g and 4°C for 1 hr, and the supernatant was collected. The supernatant was sequentially washed with a Ni-NTA column (purchased from Nanjing GenScript Biotechnology Co., Ltd.) and an MBPTrap HP column (purchased from Cytiva Biotechnology Co., Ltd.). Then, 2 µL of TEV protease (1 U/µL) was added for every 8 µg of recombinant protein to remove the MBP tag by enzymatic digestion overnight at 4°C. Finally, the final recombinant protein was purified by Superdex 200 Increase 10/300 GL column (purchased from Cytiva Biotechnology Co., Ltd.). The obtained target recombinant protein has only one additional Gly/Ser residue attached to the N-terminal, which minimizes the impact on the structure and function of the recombinant protein.

The obtained recombinant protein was stored in a buffer containing 40 mM HEPES, pH 7.5, 500 mM NaCl, and 5 % glycerol and refrigerated at -80°C until use.

Endotoxins were further removed from the purified protein solution using methods known in the art, see, for example, M. Teodorowicz et al., Optimized Triton X-114 assisted lipopolysaccharide (LPS) removal method reveals the immunomodulatory effect of food proteins. PloS one 12, e0173778 (2017); S. Liu et al., Removal of endotoxin from recombinant protein preparations. Clinical biochemistry 30, 455-463 (1997).

In addition, herein, the concentration of the protein solution may be adjusted to not more than 1 mM as described below, as necessary. Triton X-114 (purchased from Sigma-Aldrich) was added to the protein solution to a final concentration of 1% v/v. The solution with Triton X was incubated at 4°C for 60 min with constant stirring. Subsequently, the solution was transferred to a 30 °C water bath and incubated for 30 min. Then, the solution was centrifuged at 20,000 × g for 20 min at room temperature to separate the supernatant layer containing protein from the Triton X-114 layer. The supernatant layer was transferred to an endotoxin-free tube (purchased from Shanghai Shenggong Bioengineering Co., Ltd.) by pipetting.

The obtained recombinant protein was detected by SDS-PAGE as follows:
Dissolving the electrophoresis buffer powder (Tris-MOPS-SDS Running Buffer Powder, purchased from Nanjing GenScript Biotechnology Co., Ltd.) in 1 L of deionized water to prepare 1× electrophoresis buffer. SurePAGE precast gels (purchased from Nanjing GenScript Biotechnology Co., Ltd.) were prepared according to the manufacturer's instructions, and gel electrophoresis was performed using the Bio-Rad Mini-PROTEAN^{®} Tetra System5 (purchased from Bio-Rad) in 1× MOPS running buffer. After electrophoresis, the gel was stained with Coomassie Brilliant Blue using the eStain^{®} Protein Rapid Staining System (purchased from Nanjing GenScript Biotechnology Co., Ltd.) according to the manufacturer's instructions. The staining results are shown in Fig. 1.

The results showed that CN-P2S2-DN and its various truncated variants (Fig. 1A), as well as synthetic ligands targeting different tumor-associated antigens (Fig. 1B) all presented uniform electrophoretic bands with the correct molecular weight.

### Example 3: In vitro liquid-liquid phase separation experiments of synthetic ligands and their truncated variants

The purified recombinant protein CN-P2S2-DN (shown in SEQ ID NO: 24) prepared in Example 2 was used in the in vitro liquid-liquid phase separation (LLPS) performed as follows.

### (1) Fluorescently labeled recombinant protein

According to the manufacturer's instructions, the purified recombinant protein CN-P2S2-DN or its various truncated variants were mixed with fluorescent dyes Alexa Fluor^{™} 488 C₅ maleimide, Alexa Fluor^{™} 488 C₅ maleimide, or Alexa Fluor^{™} 546 C5 maleimide (all purchased from Thermo Fisher Scientific) at a 1:1 molar ratio, vortexed at room temperature and incubated for 1 hr. Then, the free dye was removed by centrifugation using Zeba^{™} Spin Desalting Columns (purchased from Thermo Scientific^{™}, Cat. No. 89882) to obtain the fluorescently labeled recombinant protein. The resulting recombinant protein was stored at -80°C until use.

### (2) In vitro liquid-liquid phase separation

The Alexa 488-labeled CN-P2S2-DN protein solution prepared as described above was inoculated on a 384 low-binding multi-well 0.17 mm microscope plate (In Vitro Scientific) at room temperature, recorded, and sealed with optically transparent film (purchased from Beijing Solebow Technology Co., Ltd.). The recombinant proteins were diluted with reaction buffer (20 mM HEPS, pH 7.4, 150 mM NaCl and 10% crowding agent PEG8000) to final concentrations of 50 µM, 25 µM, 12.5 µM, 6.35 µM, and 3.125 µM, respectively, in a total volume of 10 µl to observe the occurrence of phase separation.

### Example 4: Fluorescence confocal imaging

The microscopic plate prepared in Example 3 for inducing in vitro phase separation was placed under a NIKON A1R HD25 laser confocal microscope (purchased from Nikon, Inc.), and the CN-P2S2-DN protein droplets were subjected to fluorescence confocal imaging under a microscope with a 100x oil lens, according to a fluorescence confocal imaging method known to those skilled in the art. The induction of the in vitro liquid-liquid phase separation was observed.

For the droplets containing 12.5 µM CN-P2S2-DN protein, the initiaion time of observation was designated as 0 min, and fluorescence images were taken at specified times (0, 1, 2, 3, and 25 min) to observe the droplets and their fusion. The results are shown in Figs. 2A and 2B.

The above results show that in the in vitro liquid-liquid phase separation experiment, the CN-P2S2-DN recombinant protein can form multiple spherical droplets that are attached to the surface of the coverslip for the sake of gravity (see Fig. 2A). In time-lapse observation, the droplet-like condensates formed by CN-P2S2-DN can fuse into larger condensates through crosslinking (see Fig. 2B).

### Example 5: Fluorescence recovery after photobleaching (FRAP) assay

Fluorescence recovery after photobleaching (FRAP) is an experiment commonly used to verify liquid-liquid phase separation, see, for example, Alberti, Simon et al., Journal of Molecular Biology 430(23): 4806-4820, 2018; McSwiggen, David T et al., Genes & Development 33(23-24): 1619-1634, 2019. Therefore, FRAP was performed on the same droplets as in Example 4 that contain 12.5 µM CN-P2S2-DN protein. After bleaching with 488 nm laser light at a laser intensity of 50% for 1 second, fluorescence images were taken under a NIKON A1R HD25 laser confocal microscope (purchased from Nikon, Inc.) with a 100x oil immersion objective lens. The images were analyzed and exported using NIS-Elements AR Analysis (Nikon, Inc.) and Image J (National Institutes of Health) software.

During the entire observation time course, the time point of applying photobleaching pulse to the droplet was taken as time 0, the fluorescence intensity was recorded at each indicated time point. The mean fluorescence intensity of the photobleached area was normalized according to the mean fluorescence intensity of the unbleached area, and the fluorescence intensity graph was plotted using Graphpad Prism8 (GraphPad, Inc). The results are shown in Fig. 3. Data are the mean of three independent measurements.

The results showed that after the laser pulse ended, the average fluorescence intensity in the photobleached area gradually recovered over time, indicating that in the formed condensed phase, the CN-P2S2-DN protein molecules could still diffuse freely, allowing the fluorescence to be restored in the bleached area.

According to the results of the droplet fusion experiment and the photobleaching recovery experiment, the droplets induced by the CN-P2S2-DN recombinant protein of the present invention prepared in Example 3 have liquid-like properties and belong to liquid-liquid phase separation.

### Example 6: Intracellular liquid-liquid phase separation experiment

### (1) Phase separation based on exogenously expressed receptors

HEK293T cells were seeded at a density of 5 × 10⁵ cells/well into a 4-well chamber 35 mm culture dish (purchased from In Vitro Scientific) and cultured at 37°C, 5% CO₂ until the cell density reached approximately 60-70% confluence. Then, plasmids pCDNA3.1-CXCR4-mCherry and pCDNA3.1-DR5-GFP were gently pipetted and mixed with Opti-MEM Medium (purchased from Invitrogen, Inc.) without antibiotics and serum in a sterile centrifuge tube, in an amount of 0.5 µg pCDNA3.1-CXCR4-mCherry and 0.5 µg pCDNA3.1-DR5-GFP per well. Lipo8000^{™} transfection reagent (purchased from Shanghai Bio-Tech Biotechnology Co., Ltd.) was added and gently pipetted and mixed again to obtain a plasmid solution for transfection (i.e., each 100 µl Opti-MEM Medium contained 0.5 µg of plasmids pCDNA3.1-CXCR4-mCherry and pCDNA3.1-DR5-GFP, and 1.6 µl Lipo8000). The evenly mixed plasmid solution was added dropwise to each well, gently mixed, and then cultured for 24-36 hours at 37°C and 5% CO₂ to obtain HEK293T cells co-expressing CXCR4-mCherry and DR5-GFP.

200 nM CN-P2S2-DN protein or its different truncated variants P2S2-CN, P2S2, P2S2-DN (all labeled with fluorescent dye Alexa 647) were added to the above cell culture medium. HEK293T cells treated in the same manner with blank solution under the same conditions were used as negative controls.

Nuclear staining of HEK293T cells co-expressing CXCR4-mCherry and DR5-GFP was performed using Hoechst 33258 dye (purchased from Invitrogen. Inc) according to the manufacturer's instructions. Then, the microscopic dish was placed in the living cell culture device of a NIKON A1R HD25 laser confocal microscope. After adding the recombinant protein labeled with Alexa 647, the time sequence imaging results were immediately acquired to obtain fluorescent images corresponding to CXCR4-mCherry, DR5-GFP, the recombinant protein labeled with Alexa 647, and DAPI, respectively, as well as the co-localization images by overlaping the above fluorescent images. The results are shown in Figs. 4 to 8.

Then, for HEK293T cells treated with 200 nM CN-P2S2-DN (Alexa 647), the fluorescence recovery progress corresponding to CN-P2S2-DN (Alexa 647), CXCR4-mCherry, or DR5-GFP was measured respectively by the same method as Example 5. The results are shown in Figs. 9 to 10A.

Figs. 4 to 8 show that for HEK293T cells co-expressing CXCR4-mCherry and DR5-GFP, it was observed that GFP and mCherry signals were enriched and localized in the condensates formed by CN-P2S2-DN protein, under the addition of CN-P2S2-DN (Alexa 647). In contrast, the above colocalization and enrichment phenomenon was not observed when only the control solution or the truncated variant was added.

Fig. 9 and Fig. 10A show the spatiotemporal analysis results of the photobleaching experiment of either CN-P2S2-DN protein, CXCR4-mCherry or DR5-GFP. Both CN-P2S2-DN and CXCR4-mCherry can quickly redistribute from the unbleached area to the bleached area. For DR5-GFP, once it is recruited into the condensate containing the aforementioned three components, it exhibits relatively solid-like properties.

The above results indicate that the CN-P2S2-DN protein can not only simultaneously bind to the corresponding CXCR4 receptor and DR5 receptor via the ligands CN and DN, but also induce phase separation through the phase separation motif P2S2, resulting in driving the aggregation of unbound receptors in the surrounding area, therebybeing conducive to the oligomerization of these receptors.

### (2) Phase separation based on endogenous cell receptors

SJSA-1 cells, a human osteosarcoma cell line with known CXCR4⁺/DR5⁺, were seeded into a 4-well 35 mm culture dish (purchased from In Vitro Scientific) at a density of 5×10⁵ cells/well, and were treated for 1 hr, 30 min, 15 min, and 0 min respectively after adding 200 nM CN-P2S2-DN protein (Alex546). The cells were then fixed with 4% paraformaldehyde (Shanghai Biotech Biotechnology Co., Ltd.), permeabilized with 0.1% TritonX-100 (Shanghai Biotech Biotechnology Co., Ltd.), and blocked with 0.5% BSA/PBS (Shanghai Sangon Biotechnology Co., Ltd.) for 30 min. The cells were washed five times with 1× PBS at room temperature, 5 min each time. Then, rabbit anti-DR5-specific polyclonal antibody (purchased from Proteintech, Catalog No. 15497-1-AP) and mouse anti-CXCR4 monoclonal antibody (purchased from Proteintech, Catalog No. 60042-1-Ig) were added simutaneously as primary antibodies and incubated at 4°C overnight. The cells were washed five times with 1× PBS at room temperature, 5 min each time. Afterwards, donkey anti-rabbit IgG (H+L) Highly cross-adsorbed secondary antibody, Alexa Fluor^{™} Plus 488 (purchased from Thermo Fisher Scientific, Catalog No. A32790) and donkey anti-mouse IgG (H+L) Highly cross-adsorbed secondary antibody, Alexa Fluor^{™} Plus 568 (purchased from Thermo Fisher Scientific, Catalog No. A10037) were added as secondary antibodies and incubated at room temperature for 1 hr. The cells were washed five times with 1× PBS, 5 min each time. Then, the cells were sealed in 4',6'-diamidino-2-phenylindole (DAPI) (purchased from Beyotime), and immunofluorescence was observed and photographed using a NIKON super-resolution microscope-SIM/STORM. The results are shown in Fig. 10B.

SJSA-1 cells, a human osteosarcoma cell line known to be CXCR4⁺/DR5⁺, were seeded into 4-well chamber 35 mm culture dishes (purchased from In Vitro Scientific) at a density of 5×10 ⁵ cells/well. Cells were cultured at 37°C, 5% CO₂ until the cell density reaches about 60-70% confluence. Then, 200 nM CN-P2S2-DN protein with Alexa 561 fluorescence labeling was added to the culture medium, and the cells were incubated at 37°C and 5% CO₂ for 4-6 hr. The snapshots of the SJSA-1 cells treated as described above were taken by A NIKON A1R HD25 laser confocal microscope (purchased from Nikon, Inc.) with a 100× oil immersion objective lens. The results are shown in Fig. 11A.

The above results show that CN-P2S2-DN can also bind to the corresponding endogenous receptors in DR5⁺/CXCR4⁺ double-positive SJSA-1 cells, thereby aggregating on the cell surface to form a micron-sized puncta structure that is unique to liquid-liquid phase separation.

Microscopic observation was performed on specific spot structures. The moment of adding CN-P2S2-DN protein into the culture medium fortreating the cells was designated as time 0. Time sequence imaging was performed on the same specific area on the SJSA-1 cell surface at 0 min, 0.5 min, 1 min, and 1.5 min, respectively. The observed results are shown in Fig. 11B. The results showed that the spots also exhibited the same contact fusion phenomenon as those observed in the in vitro phase transition droplets.

### Example 7: Co-localization of recombinant protein and downstream effector molecules

HEK293T cells were seeded at a density of 5 × 10⁵ cells/well into a 4-well chamber 35 mm culture dish (purchased from In Vitro Scientific) and cultured at 37°C until the cell density reached approximately 70-80% confluence.

Exogenous CXCR4-mCherry, DR5-GFP, and FADD-BFP were simultaneously transfected into the cell culture medium, and 200 nM CN-P2S2-DN recombinant protein (labeled with Alexa 647) was added 24 hr after transfection. The cells were incubated at 37°C and 5% CO₂ for 30 min, and immunofluorescence was observed and photographed using a NIKON A1R HD25 laser confocal microscope. The results are shown in Fig. 27A. The fluorescence intensity at the straight line with a white arrow in the figure was measured using the same software and parameters as in Example 5 for fluorescence image photographing. The results are shown in Fig. 27B.

Figs. 27A and 27B show that in cells treated with recombinant protein, FADD (as a downstream effector in the DR5 cascade signaling pathway) was co-localized with DR5 aggregates, suggesting that the recombinant protein can normally recruit effector proteins through the DR5 receptor and initiate the downstream signaling cascade reaction.

### Example 8: Colocalization of recombinant proteins and endogenous receptors

SJSA-1 cells with known CXCR4⁺/DR5⁺ were seeded at a density of 5 × 10⁵ cells/well into a 4-well chamber 35 mm culture dish (purchased from In Vitro Scientific) and cultured at 37°C until the cell density reached approximately 70-80% confluence.

200 nM CN-P2S2-DN recombinant protein and its truncated variants P2S2 and P2S2-DN (both labeled with Alexa 647) were added into the cell culture medium. Cells were icubated at 37°C, 5% CO₂ for 4-6 hr. Then, the cells were fixed with 4% paraformaldehyde (Shanghai Bio-Tech Biotechnology Co., Ltd.), permeabilized with 0.1% TritonX-100 (Shanghai Bio-Tech Biotechnology Co., Ltd.), and blocked with 0.5% BSA/PBS (Shanghai Sangon Biotechnology Co., Ltd.). The cells were washed five times with 1× PBS at room temperature, 5 min each time. Then, rabbit anti-DR5-specific polyclonal antibody (purchased from Proteintech, Catalog No. 15497-1-AP) and mouse anti-CXCR4 monoclonal antibody (purchased from Proteintech, Catalog No. 60042-1-Ig) were added simultaneously as primary antibodies and incubated at 4°C overnight. The cells were washed five times with 1× PBS at room temperature, 5 min each time. Afterwards, donkey anti-rabbit IgG (H+L) Highly cross-adsorbed secondary antibody, Alexa Fluor^{™} Plus 488 (purchased from Thermo Fisher Scientific, Catalog No. A32790) and donkey anti-mouse IgG (H+L) Highly cross-adsorbed secondary antibody, Alexa Fluor^{™} Plus 568 (purchased from Thermo Fisher Scientific, Catalog No. A10037) were added as secondary antibodies and incubated at room temperature for 1 hr. The cells were washed five times with 1× PBS, 5 min each time. Then, the cells were sealed in 4',6'-diamidino-2-phenylindole (DAPI) (purchased from Beyotime), the immunofluorescence was observed and photographed using a NIKON A1R HD25 laser confocal microscope. The results are shown in Figs. 12A, 13A, and 28A. For the captured fluorescence images, the fluorescence intensity at the straight lines with white arrows in Figs. 12A, 13A, and 28A was measured using the same software and parameters as in Example 5. The results are shown in Figs. 12B, 13B, and 28B.

The above results showed that compared with the truncated variants, only the recombinant protein CN-P2S2-DN can co-localize with the endogenous receptors of the cells, and the co-localization pattern was almost consistent with the pattern observed in the extracellular experiment in Example 3.

### Example 9: Photobleaching recovery of recombinant proteins in tumor cells

SJSA-1 cells were treated with 200 nM CN-P2S2-DN (labeled with Alexa 561) for 4 hrs in the same manner as in Example 5, and photobleaching analysis was performed. The results are shown in Fig. 14.

The results of FRAP experiment showed that CN-P2S2-DN specifically bound to the endogenous CXCR4 receptor and DR5 receptor of tumor cells and induced the formation of phase separation-driven co-aggregation spots with liquid-like dynamic properties. This means that CN-P2S2-DN protein can induce phase separation based on the binding of phase separation motif P2S2 with the endogenous receptors on the cell membrane surface via two ligands CN and DN, resulting in driving the aggregation of unbound endogenous receptors in the surrounding cell membrane region, thereby being conducive to the oligomerization of these receptors.

### Example 10: Modification of inter-receptor communication by recombinant proteins

The manipulation activities of the various recombinant proteins prepared in Example 2 on the downstream signal proteins of the receptor were analyzed by cell morphology observation and Western blotting.

### (1) Cell morphological changes

SJSA-1 cells with known CXCR4⁺/DR5⁺ were seeded at a density of 5 × 10⁵ cells/well into a 4-well chamber 35 mm culture dish (purchased from In Vitro Scientific) and cultured at 37°C until the cell density reached approximately 70-80% confluence. 100 nM CN-P2S2-DN, its truncated variants (P2S2-CN, P2S2-DN, P2S2, CN, DN, CN-DN), or combinations of variants (combination of P2S2-CN and P2S2-DN, combination of CN and DN) were added to the cell culture medium, and the cells were incubated at 37°C, 5% CO₂ for 3 hr. Cells treated with corresponding blank buffer were used as solution control. Living cells were photographed using an Olympus IX83 fluorescence inverted microscope, and the results are shown in Fig. 15A.

SJSA-1 cells were seeded into 4-well 35 mm culture dishes at a density of 5 × 10⁵ cells/well and cultured at 37°C until the cell density reached approximately 70-80% confluence. 100 nM CN-P2S2-DN and green fluorescent carbocyanine nucleic acid stain YO-PRO (Thermo Fisher Scientific, 1:2000 dilution) were added to the cell culture medium, and the cells were incubated at 37°C, 5% CO₂ for 3 hr. It is known that the cell membrane permeability of apoptotic cells increases, allowing YO-PRO to enter the cell and bind to DNA to develop color. Time-lapse confocal photography was performed at the indicated time points using a NIKON A1R HD25 confocal microscope equipped with a 100× oil immersion objective. The results are shown in Fig. 15B.

Under different CN-P2S2-DN addition concentrations, the ratio of YO-PRO fluorescence-positive cells was measured over time. The results are shown in Fig. 15C.

The above results showed that compared with various truncated variants, variant combinations and solution controls, a series of significant morphological changes were only observed in SJSA-1 cells incubated with CN-P2S2-DN, such as shrinkage, increase of cell membrane permeability, and rapid phagocytosis by neighboring cells. Obvious cell death can be observed at a concentration as low as 1 nM CN-P2S2-DN, and the apoptosis-inducing effect is time-dependent and concentration-dependent.

### (2) Changes in signal transduction pathways

Activation mediated by cleavage of full-length caspase 3 is a key event in the process of apoptosis (see, e.g., Nicholson, Donald W. et al., "Identification and inhibition of the ICE/CED-3 protease necessary for mammalian apoptosis." Nature 376.6535 (1995): 37-43; Hsia, Jiun-Yi et al., "Prognostic significance of caspase-3 expression in primary resected esophageal squamous cell carcinoma." European Journal of Surgical Oncology (EJSO) 29.1 (2003): 44-48). The Western blotting method commonly known in the art was used to analyze the changes in the expression level of pro-Cysteine proteases 3 (pro-caspase-3) in SJSA-1 cells after the addition of the recombinant protein of the present invention. See, for example, Zhang, T. et al., "Discovery of a novel third-generation EGFR inhibitor and identification of a potential combination strategy to overcome resistance." Molecular Cancer 19.1 (2020).

Specifically, endotoxin-free CN-P2S2-DN recombinant protein, its truncated variants (P2S2-CN, P2S2-DN, P2S2, CN, DN, CN-DN), or combinations of variants (combination of P2S2-CN and P2S2-DN, combination of CN and DN) were added to the culture medium of SJSA-1 cells (1×10⁶ cells/well) at a final concentration of 100 nM and incubated at 37°C for 3 hr. Then, SJSA-1 cell lysate was prepared using Minute^{™} Total Protein Extraction Kit for Animal Cultured Cells/Tissues (purchased from Invent Biotech) according to the manufacturer's instructions. The lysate was resuspended in 2× Laemmli loading buffer (purchased from Sigma-Aldrich, Inc). By the same method as Example 2, the protein sample was transferred to a PVDF membrane via SDS-PAGE. After transfer, the PVDF membrane was washed several times with deionized water, and then a blocking solution (TBST containing 5% skim milk) was added and blocked at room temperature for 2 hr. The blocking solution was discarded, and rabbit anti-Caspase-3 polyclonal antibody (purchased from CST, Cat. No. 9662) diluted in the blocking solution was added as the primary antibody, and incubated at 4°C overnight. Then, the primary antibody solution was discarded and the membrane was washed five times with TBST, each time for 6 min. Then, F(ab')₂ -goat anti-rabbit IgG (H+L), HRP was used as the secondary antibody and incubated at room temperature for 1 hr. Finally, protein bands were visualized using enhanced chemiluminescence reagents. The results are shown in Fig. 16A. The results showed that only the addition of CN-P2S2-DN recombinant protein significantly reduced the level of full-length caspase 3 in SJSA-1 cells, in comparison with the additions of anyone of the truncated variants, variant combinations and blank control.

In addition, endotoxin-free CN-P2S2-DN recombinant protein was added to the culture medium of SJSA-1 cells and HEK293T cells at final concentrations of 0, 50, 100, 200, and 500 nM, respectively, and the expression level of pro-caspase 3 in cells was determined by Western blotting using rabbit anti-Caspase-3 polyclonal antibody (purchased from CST, Cat. No. 9662) as the primary antibody. The results are shown in Fig. 16B. The results showed that in SJSA-1 cells with known CXCR4⁺/DRS⁺, the content of caspase-3 was significantly reduced in a CN-P2S2-DN recombinant protein concentration-dependent manner, indicating that caspase-3 was significantly activated in the cells. In contrast, no activation of caspase 3 was detected in the non-tumor cell line HEK293T.

From the above results, it was confirmed that CN-P2S2-DN of the present invention strongly forms aggregates based on ligand-receptor binding and can regulate communication between endogenous receptors as a multivalent binary system.

According to the manufacturer's instructions, Caspase-Glo^{®} 3/7 Kit (purchased from Promega, Catalog No. G8090) and Caspase-Glo^{®} 8 Kit (purchased from Promega, Catalog No. G8200) were used to measure the luciferase luminescence signal intensity of SJSA-1 cells treated with 100 nM endotoxin-free CN-P2S2-DN recombinant protein as described above by a SPARK microplate reader (TECAN), at 0.5, 1, 3, 6, 10, and 24 hours after the addition of the recombinant protein. The results are shown in Figs. 17A and 17B. The above results showed that the activities of caspase 3/7 and caspase 8, which are closely related to cell apoptosis, increased over time in SJSA-1 cells treated with the recombinant proteins and reached a peak at 3 hours after treatment.

### Example 11: Induction of apoptosis in synthetic cells by recombinant protein CN-P2S2-DN

In order to confirm that the phase transition adjusting element of the present invention promotes phase separation based on binding to cell surface DR5 receptors and tumor-associated antigens, thereby regulating tumor cell apoptosis, this example mainly analyzes the apoptosis-inducing activity of the recombinant protein CN-P2S2-DN prepared in Example 2 and its various truncated variants (P2S2-CN, P2S2-DN, P2S2, CN, DN, CN-DN, P2-CN, S2-DN) and the combination of truncated variants (the combination of P2S2-CN and P2S2-DN, the combination of CN and DN, the combination of P2-CN and S2-DN) on the tumor cell line SJSA-1 expressing DR5 by flow cytometry. As negative controls, blank solution and non-tumor HEK293T cells were used.

### (1) Apoptosis-inducing activity against SJSA-1 cells, COLO206 cells, and 293T cells

SJSA-1 cells were seeded at 1×10⁵ cells/well in a 96-well plate (Falcon), and the prepared endotoxin-free CN-P2S2-DN protein was added to the cell culture medium to a final concentration of 100 nM, and incubated at 37°C for 4-6 hr. Then, SJSA-1 cells were labeled with CellTracker^{™} Green CMFDA Dye (purchased from Invitrogen, Inc.) according to the manufacturer's instructions, and cell proliferation was monitored using the Incucyte^{®} Zoom System (purchased from Essen, USA), and the cell growth status was recorded and analyzed using the Incucyte^{®} Analysis software. The results are shown in Fig. 18. The results showed that at a dose of 100 nM, cell death was observed as early as 3 hr after the addition of the recombinant protein and reached a plateau 8 hr after the addition.

SJSA-1 cells, COLO206 cells, and 293T cells were seeded at 1 x 10⁶ cells/well in 24-well plates (Falcon), and the prepared recombinant protein CN-P2S2-DN and the truncated variants and combinations of variants were added at a final concentration of 100 nM, respectively, and reacted at 37°C, 5% CO₂ for 3 hr. After the cells were washed with PBS buffer, Annexin V-FITC/PI double staining was performed using the Annexin V-FITC Cell Apoptosis Detection Kit (purchased from Beyotime) according to the manufacturer's instructions, and the fluorescence intensity was measured using a flow cytometer LSRFortessa SORP (BD Company) to determine the apoptotic ratio of SJSA-1 cells. The results are shown in Figs. 19A to 19C.

The results in Fig. 19 showed that in CXCR⁺/DRS⁺ SJSA-1 cells and COLO-205 cells, CN-P2S2-DN can induce significantly higherratio of Annexin V-positive apoptotic cells than not only the blank solution ( the negative control), but also the truncated variant CN-DN linked by known linker technology, the truncated variant combination CN+DN without a linker therebetween, and all other truncated variants and combinations of variants. Notably, no obvious apoptosis was observed in SJSA-1 cells supplemented with a combination of the truncated variants P2-CN and S2-DN. This suggested that the adjusting element of the present invention (which derived from tandemly linking the motifs essential for phase separation together), can significantly improve the phase separation effect compared to the kit form in which the motifs exist separately, thereby enhancing the function of the element in regulating the activity of cell surface molecules.

### (2) Comparison of apoptosis-inducing activity among SJSA-1 cells, COLO-205 cells, and HEK293T cells

Based onthe same method as in Example (1), the apoptosis-inducing activity of 100 nM recombinant protein CN-P2S2-DN on tumor cells SJSA-1, COLO-205 and non-tumor cells HEK293T was determined under the same conditions. Meanwhile, the expression level of DR5 in SJSA-1 cells, COLO-205 cells and HEK293T cells was determined using the Q-PCR method well known in the art. See, e.g., VanGuilder, Heather D et al., Biotechniques 44(5): 619-626, 2008. The results of apoptosis induction are shown in Fig. 20.

Fig. 20 shows that the apoptosis ratio induced by CN-P2S2-DN in SJSA-1 tumor cells was 82.5%, and the apoptosis ratio induced by CN-P2S2-DN in COLO-205 cells was 74%, both of which were significantly higher than those of HEK293T cells (9.4%) and blank controls (4.6%). On the other hand, there was no statistically significant difference between HEK293T cells and the blank control group.

CN-P2S2-DN shows significantly different apoptosis-inducing effects in tumor cell lines and non-tumor cell lines, which may be due to the significant difference in the expression levels of cell surface receptor molecules between the two types of cell lines. According to the manufacturer's instructions, the expression of CXCR4 and DR5 on the cell membrane surface was determined using a PE fluorescent quantitative kit (purchased from BD Quantibrite, catalog number 340495) and corresponding antibodies (generally diluted 1:20), and flow cytometry analysis was performed using a BD LSRFortessa SORP instrument to determine the expression level of cell surface receptors. As shown in Fig. 21, unlike the known CXCR4⁺/DRS⁺ double-positive tumor cell lines SJSA-1 and COLO-205, the non-tumor cell line HEK293T lacks DR5 expression and barely expresses CXCR4 (see Fig. 21). This suggests that interaction through recognition and binding to cell surface molecules (such as specific ligands) is critical for the recombinant fusion protein of the present invention to manipulate cell surface molecule-related cellular events.

### (3) Activity concentration curve of recombinant protein CN-P2S2-DN

Flow cytometry was used in the same way to evaluate the activity of CN-P2S2-DN recombinant protein at different concentrations to induce cell apoptosis. A series of different concentrations of endotoxin-free CN-P2S2-DN recombinant protein solutions were prepared and added to SJSA-1 cells (1×10⁶ cells/well), incubated at 37°C for 4-6 hr. The apoptotic ratio of SJSA-1 cells under different concentrations was determined as described above, and a dose-response curve was drawn. The results are shown in Figs. 22A and 22B. The results showed that CN-P2S2-DN exhibited strong apoptosis-inducing activity in both CXCR4⁺/DRS⁺ SJSA-1 cells and COLO-205 cells, with IC₅₀ values of 10.14 nM and 28.91 nM, respectively.

### (4) Apoptosis-inducing activity against other tumor cells

Using the same method, the apoptosis-inducing activity of CN-P2S2-DN recombinant protein or its truncated variants P2S2-DN and P2S2 in other double-positive tumor cell lines expressing *CXCR4* and *DR5* genes, including NCI-H226, NCI-H1975, MDA-MB-231, PC-3, A549, and HepG2. A blank solution was used as a negative control, and the results are shown in Fig. 23.

As a result, no difference in apoptosis rate was observed between the non-tumor cell line HEK293T cells and the control group. On the other hand, CXCR4⁺/DRS⁺ double-positive tumor cells (including colorectal, sarcoma, lung and prostate cell lines) all showed significant responsiveness to CN-P2S2-DN, with apoptosis ratio Ratioₘᵢₙ > 40%. Therefore, it is confirmed that there is no dependence on specific tumor cell types for the phase transition adjusting element of the present invention in view of their manipulation of cell surface molecules and other related functions, the element is effective in a variety of cancer cell lines.

### Example 12: Activity of recombinant protein CN-S2S2-DN

Using another recombinant protein CN-S2S2-DN (as shown in SEQ ID NO: 25), the same method as in Examples 3 to 5 was repeated to further confirm the control activity of the phase transition adjusting fusion protein of the present invention on cell apoptosis through phase separation. The results confirmed that although different multivalent phase transition domains were used, the recombinant protein CN-S2S2-DN was also able to form multiple spherical droplets in vitro characterized in liquid-liquid phase separation, and exhibited typical photobleaching recovery in FRAP experiments. This indicates that the recombinant protein can also effectively induce phase separation.

The apoptosis-inducing activity of the recombinant protein CN-S2S2-DN against SJSA-1 cells and HEK293T cells was further compared using flow cytometry analysis in the same manner as in Example 11. The results are shown in Fig. 24. The results showed that the apoptosis-inducing activity of CN-S2S2-DN was significantly different between SJSA-1 cells (21.6%) as tumor cells and HEK293T cells (2.3%) as non-tumor cells, indicating that changing the multivalent phase transition domain did not have a substantial effect on the activity of the phase transition adjusting element of the present invention in manipulating cell surface molecules (such as inducing tumor cell apoptosis).

### Example 13: Activity of low-valent recombinant proteins or mutant recombinant proteins

Using the same method as in Examples 1 and 2, purified low-valent recombinant protein CN-P1S1-DN (as shown in SEQ ID NO: 27) and mutant recombinant protein CN-P2S2-DN MUT (as shown in SEQ ID NO: 28) were obtained.

The cells were seeded at a density of 1 × 10 ⁴ cells/well in a 96-well plate and incubated at 37 °C for 16 h to 70% confluence. Then, the mutant recombinant proteins were added at the indicated concentrations (1, 10, and 100 nM) and treated at 37°C for 3 h. Cell viability was determined using the Cell Titer Glo^{®} Luminescent Cell Viability Assay (Promega) according to the manufacturer's instructions. The luminescence signal was measured using a SPARK luminometer (TECAN). The results are shown in Fig. 29. Fig. 29 shows that the introduction of mutations that can disrupt the interaction between multivalent phase transition domains (especially proline to alanine) will reduce the efficacy of the recombinant protein of the present invention in inducing tumor cell apoptosis. This also confirms that phase separation mediated by multivalent phase transition domains is a key factor in driving conditional apoptosis of tumor cells.

Using the same method as in Example 11 (1), the growth status of SJSA-1 and COLO-205 cells under the condition of adding 100 nM endotoxin-free CN-P2S2-DN or CN-P1S1-DN was measured. The results are shown in Figs. 30A and 30C, respectively. The results show that in both tumor cells, the higher-valent CN-P2S2-DN had significantly higher apoptosis-inducing activity than the lower-valent CN-P1S1-DN.

The same method as in Example 11 (3) was used to determine the apoptosis ratio of SJSA-1 and COLO-205 cells under different concentration conditions, and dose-response curves were drawn. The results are shown in Figs. 30B and 30D. The results show that CN-P1S1-DN could exhibit a certain apoptosis-inducing activity in both CXCR4⁺/DR5⁺ tumor cells, and its IC₅₀ in SJSA-1 cells was 16.95 nM.

### Example 14: Induction of Synthetic Cell Apoptosis by Other Recombinant Proteins

It has been reported that a variety of tumor-associated antigens (TAAs) are expressed significantly higher on cancer cells than on normal tissues, including but not limited to hepatocyte growth factor receptor (also referred to herein as c-Met, HGFR), epidermal growth factor receptor (EGFR), etc.

According to the method for constructing synthetic ligands as shown in Example 1, the apoptosis-inducing activity of multivalent binary recombinant ligands in different tumor cell lines was evaluated, which targeted different tumor-associated antigens and cell surface receptors. Each recombinant ligand and its truncated variant used was prepared using the methods described in Examples 1 and 2.

### (1) Recombinant protein EN-P2S2-DN

The recombinant protein EN-P2S2-DN was prepared and purified according to the same method as Example 2. EN-P2S2-DN is a synthetic ligand obtained by covalently linking anti-EGFR nanobody (also referred to as EN herein, SEQ ID NO: 4) and anti-DR5 nanobody at the terminalsof P2S2.

SJSA-1, COLO205, NCI-H1975, HepG2, and Hela cells were used as examples of tumor cells with expression of the tumor-associated antigen EGFR on their surfaces. The expression of EGFR was verified by flow cytometry. The aforementioned cell lines were seeded at 1 x 10⁶ cells/well in 24-well plates (Falcon), and endotoxin-free EN-P2S2-DN recombinant protein was added to 500 nM, and the reaction was carried out at 37°C and 5% CO₂ for 5 hr. Then, the apoptosis-inducing activity of EN-P2S2-DN in different cell lines was determined by flow cytometry analysis based on the Annexin V-FITC/PI double staining method (Beyotime) in the same manner as in Example 11. Non-tumor HEK 293T cells were used as negative control. The results are shown in Fig. 25.

The results showed that all EGFR⁺/DR5⁺ double-positive tumor cells showed significant responsiveness to EN-P2S2-DN, among which the greatest response was observed in the human cervical cancer cell line HeLa. On the other hand, only 1.2% of non-tumor HEK293T cells were observed to be apoptotic, which was a significantly low proportion. Therefore, it was confirmed that EN-P2S2-DN can induce tumor cell apoptosis by manipulating cell surface receptor-mediated signaling pathways.

### (2) Recombinant protein MN-P2S2-DN

The recombinant protein MN-P2S2-DN was prepared and purified according to the same method as Example 2. MN-P2S2-DN is a synthetic ligand obtained by covalently linking anti-c-Met nanobody (also referred to as MN herein, SEQ ID NO: 5) and anti-DR5 nanobody at the terminals of P2S2.

Six different tumor cell lines (osteosarcoma cell line SJSA-1, colon adenocarcinoma cell line COLO-205, lung squamous cell carcinoma cell line NCI-H226, lung squamous cell carcinoma cell line NCI-H1975, liver cancer cell line HepG2, cervical cancer cell line Hela) were used as examples of tumor cells with expression of the tumor-associated antigen c-Met on surfaces. The expression of c-Met was verified by flow cytometry. The apoptosis-inducing activity of MN-P2S2-DN in the aforementioned cell lines was determined by the same method as above. Non-tumor HEK 293T cells were also used as negative control. The results are shown in Fig. 26.

The results showed that c-Met⁺/DR5⁺ double-positive tumor cells showed significant responsiveness to MN-P2S2-DN, with an apoptosis rate of about 15% to about 60%. On the other hand, in the non-tumor cell line HEK293T cells, the apoptosis ratio was only 1.3%, which was significantly lower than that of all tumor cell lines. Therefore, it was confirmed that MN-P2S2-DN can also induce tumor cell apoptosis based on the manipulation of cell surface receptor-mediated signaling pathways.

The above two different recombinant fusion proteins further confirmed that the phase transition adjusting element of the present invention manipulates the activity of cell surface molecules (such as inducing tumor cell apoptosis) and is also independent of specific ligands. Those skilled in the art can expect that phase transition adjusting elements obtained by combining with ligands of other cell surface molecules can also achieve similar control effects on cellular events.

### Example 15: Surface receptor internalization inhibitors enhance the induction of synthetic cell apoptosis by recombinant proteins

It has been reported that cellular internalization can regulate the number and density of cell surface receptors, thereby affecting the functional activity regulated by the receptor (Calebiro D, Godbole A, Internalization of G-protein-coupled receptors: implications for receptor function, in "Handbook of Experimental Pharmacology," Springer, 2014, 219-233.).

Rabbit anti-DR5-specific polyclonal antibody (purchased from Proteintech, Catalog No. 15497-1-AP) and mouse anti-Rab5 monoclonal antibody (purchased from Cell Signaling Technology, Catalog No. 46449) were used as primary antibodies, and the colocalization of DR5 and CN-P2S2-DN with the early endosomal marker Rab5 was measured 15 and 30 minutes after the addition of 100 nM CN-P2S2-DN, according to the same fluorescence colocalization and grayscale value statistical methods as in Example 6. The results are shown in Fig. 31.

Furthermore, SJSA-1 and COLO-205 cells were pretreated with a medium containing 250 mM sucrose for 60 minutes, respectively. CN-P2S2-DN was added to the cells to 10 nM and incubated at 37°C. The cell membrane surface content of cell surface DR5 was measured immediately before the addition of CN-P2S2-DN (0 min), 15, 30, and 60 min using a PE fluorescence quantification kit (purchased from BD Quantibrite, catalog number 340495) in the same manner as in Example 11. The same SJSA-1 and COLO-205 cells without sucrose pretreatment were used as controls. The results are shown in Fig. 32.

Figs. 31 and 32 show that after CN-P2S2-DN treatment, the co-localization of DR5 and CN-P2S2-DN with Rab5 was observed to increase over time, indicating that the recombinant protein caused the internalization of cell surface DR5 receptors. After 60 minutes of CN-P2S2-DN treatment, the expression of DR5 on the surface of SJSA-1 and COLO-205 cells was significantly reduced to 38.3% and 20.6%, respectively. In contrast, pretreatment with the pan-internalization inhibitor sucrose can effectively inhibit DR5 internalization and upregulate the expression of surface DR5 in SJSA-1 and COLO-205 cells to 50.5% and 35.8%, respectively.

CN-P2S2-DN at 10 nM or DR5 ligand protein TRAIL at 0.1 nM (SJSA-1 cells) or 0.01 nM (COLO-205 cells) were added to SJSA-1 and COLO-205 cells pretreated with medium containing 250 mM sucrose for 60 minutes or to cells without the above pretreatment, respectively. The cells were incubated at 37°C for 48 hr, and the cell viability was measured using the CellTiter-Glo^{®} Luminescent Cell Viability Kit (purchased from Promega, Cat. No. G7571) and a SPARK microplate reader (TECAN). The results are shown in Fig. 33.

Fig. 33 shows that pre-treatment of cells with a receptor internalization inhibitor (such as sucrose) can significantly increase the sensitivity of tumor cells to the phase transition adjusting element of the present invention, significantly enhance the activity of the element of the present invention in inducing tumor cell apoptosis, and has the potential to be used as a synergistic therapeutic agent. It should be noted that in different tumor cells, the enhancing effect of receptor internalization inhibitors on the activity of the element of the present invention is much greater than its effect when combined with the DR5 ligand TRAIL, which proves that the phase transition conditional element of the present invention induces cell apoptosis in a different way from traditional receptor ligands and is a new type of adjusting element.

The present invention has been described in detail above. It is obvious to those skilled in the art that the present invention can be implemented in a wider range under equivalent parameters, concentrations and conditions without departing from the spirit and scope of the present invention and without unnecessary experiments. While the present invention has been described in detail with reference to specific embodiments, it will be appreciated that the invention is capable of further modifications. In short, according to the principles of the present invention, this application is intended to include any changes, uses or improvements to the present invention, including changes that depart from the scope disclosed in this application and are made using conventional techniques known in the art. Some essential features may be applied within the scope of the following claims.

## Claims

1. A phase transition adjusting element, **characterized in that** a part of the phase transition adjusting element comprises a multivalent phase transition domain, and the remaining comprises at least two ligands, for example, 2, 3, 4, 5, 6, 7, 8, 9, or 10 ligands, wherein at least one ligand is linked to the part containing the multivalent phase transition domain, and the remaining ligands are linked to the part containing the multivalent phase transition domain or to another ligand contained in the same phase transition adjusting element, and each of the at least two ligands can specifically bind to a cell surface molecule corresponding thereto, preferably, the part comprising multivalent phase transition domain and the at least one ligand that contained in the same phase transition adjusting element are covalently connected via a linker such as a peptide linker or a non-peptide linker,
wherein, the cell surface molecule may be identical or different,
preferably, the phase transition adjusting element is a fusion protein.

2. The phase transition adjusting element of claim 1, wherein the multivalent phase transition domain is composed of at least two, such as three, four, five, six, seven, eight, nine or ten motifs linked tandemly.

3. The phase transition adjusting element of any one of the preceding claims, wherein the multivalent phase transition domain is composed of at least one SUMO3 motif tandemly linked with at least one SIM motif, or is composed of at least one PRMH motif tandemly linked with at least one SH3 motif.

4. The phase transition adjusting element of any one of the preceding claims, wherein the multivalent phase transition domain is composed of one, two, three or four SUMO3 motifs and one, two, three or four SIM motifs linked tandemly, or is composed of one, two, three or four PRMH motifs and one, two, three or four SH3 motifs linked tandemly, more preferably, the multivalent phase transition domain comprises the amino acid sequence shown in SEQ ID NO: 18 or SEQ ID NO: 19 or comprises an amino acid sequence that is at least 80%, 90%, 95%, 99% identical to the sequence shown in SEQ ID NO: 18 or SEQ ID NO: 19.

5. The phase transition adjusting element of any one of the preceding claims, wherein among the at least two cell surface molecules specifically bound by the at least two ligands, one cell surface molecule is a tumor-associated antigen, and preferably the tumor-associated antigen is selected from the group consisting of CXC motif chemokine receptor 4 (CXCR4), hepatocyte growth factor receptor (c-Met or HGFR), epidermal growth factor receptor (EGFR), human epidermal growth factor receptor 2 (HER2) and prostate-specific membrane antigen (PSMA), fibroblast activation protein (FAP), carcinoembryonic antigen (CEA), folate receptor alpha (FolR1), melanoma-associated chondroitin sulfate proteoglycan (MCSP), p95HER2, EpCAM, HER3, CD30 or TPBG (5T4), CD19, CD79b, CD20, CD22, CD37, CD38, BCMA and GPRC5D; and/or the other cell surface molecule is a tumor necrosis factor receptor, preferably a death receptor 5 (DR5) or a Fas receptor, more preferably a death receptor 5 (DR5).

6. The phase transition adjusting element of any one of the preceding claims, wherein the ligand may be selected from a peptide ligand or a non-peptide ligand, and the peptide ligand is preferably selected from one or more of the group consisting of: an antibody or an antigen-binding fragment thereof, a cytokine, a growth factor, an adhesion molecule, a peptide hormone, or a polypeptide randomly selected by phage display, yeast display or the like that specifically binds to a cell surface molecule; the antibody may be selected from a monoclonal antibody, a polyclonal antibody, a human antibody or a humanized antibody; the antigen-binding fragment may be selected from F(ab')₂, Fab, a single-chain variable fragment (scFv), a single-domain antibody fragment (VHH or nanobody); and the non-peptide ligand is preferably selected from one or more of the group consisting of a small molecule agonist or antagonist, an antisense oligonucleotide or a small interfering RNA (siRNA).

7. The phase transition adjusting element of any one of the preceding claims, wherein at least two ligands contained in the phase transition adjusting element are single-chain variable fragments, and preferably, one of the at least two ligands is a single-chain variable fragment comprising an amino acid sequence shown in SEQ ID NO: 21 or an amino acid sequence having at least 80%, 90%, 95%, 99% identity to the sequence shown in SEQ ID NO: 21, and/or the other ligand is a single-chain variable fragment comprising an amino acid sequence selected from SEQ ID NO: 20, SEQ ID NO: 22 or SEQ ID NO: 23 or an amino acid sequence having at least 80%, 90%, 95%, 99% identity to the sequence shown in SEQ ID NO: 20, SEQ ID NO: 22 or SEQ ID NO: 23.

8. The phase transition adjusting element of any one of the preceding claims, further comprising a tag that does not affect the functions of the ligand and the part comprising the multivalent phase transition domain, and/or a polypeptide that cleaves the tag.

9. The phase transition adjusting element of any one of the preceding claims, comprising, or consisting of, the amino acid sequence shown in SEQ ID NO: 24 or SEQ ID NO: 25, or an amino acid sequence having least 80%, 90%, 95%, or 99% identity to the amino acid sequence shown in SEQ ID NO: 24 or SEQ ID NO: 25.

10. A method for screening phase transition adjusting element, the method includes the following steps:
Step A: generating a library comprising a plurality of candidate phase transition adjusting elements, wherein each of the candidate phase transition adjusting elements comprises a part comprising a multivalent phase transition domain, and the remaining comprising at least two ligands, wherein at least one ligand is covalently linked to the part comprising the multivalent phase transition domain, and the remaining ligands are covalently linked to the part comprising the multivalent phase transition domain in the same candidate phase transition adjusting element or to other ligands contained in the same phase transition adjusting element, and each of the at least two ligands can specifically bind to a cell surface molecule corresponding thereto, respectively, and optionally, the part comprising the multivalent phase transition domain and the at least one ligand that contained in the same phase transition adjusting element, or the ligands contained in the same phase transition adjusting element, are covalently linked via a linker such as a peptide linker, wherein each of the cell surface molecule specifically bound by the ligand in one candidate phase transition adjusting element can be the same or different;
Step B: using a reference that does not contain the multivalent phase transition domain as a negative control, and measuring the activity level of the candidate phase transition adjusting element and the negative control in generating phase transition droplets under the same conditions suitable for phase transition; and
Step C: selecting the candidate phase transition adjusting element that produces more phase transition droplets than the negative control in step B as the target phase transition adjusting element.

11. A pharmaceutical composition comprising the phase transition adjusting element according to any one of the preceding claims, and optionally a pharmaceutically acceptable carrier.

12. A product comprising (a) a phase transition adjusting element according to any one of claims 1 to 9, and (b) a receptor internalization inhibitor, for simultaneous, separate or sequential use as a combined preparation in the treatment of a disease.

13. A method for treating a disease, comprising administering the phase transition adjusting element, pharmaceutical composition or product of any one of the preceding claims to an individual in need thereof, wherein the disease can be selected from the group consisting of chronic autoimmune disorders, inflammatory disorders, diseases associated with abnormal cell proliferation or abnormal cell apoptosis, sepsis or viral infection, preferably the disease associated with abnormal cell proliferation or abnormal cell apoptosis is selected from cancer, more preferably the cancer is selected from lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid cancer) or prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt's lymphoma or mycosis fungoides (MF), or metastatic forms thereof.

14. Use of the phase transition adjusting element, pharmaceutical composition or product according to any one of the preceding claims in manufacturing a preparation for treating a disease, wherein the disease can be selected from the group consisting of chronic autoimmune disorders, inflammatory disorders, diseases associated with abnormal cell proliferation or abnormal cell apoptosis, sepsis or viral infection, preferably the disease associated with abnormal cell proliferation or abnormal cell apoptosis is selected from cancer, more preferably the cancer is selected from lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid cancer) or prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt's lymphoma or mycosis fungoides (MF), or metastatic forms thereof.
